# EUROPEAN PATENT APPLICATION

(11) **EP 2 305 816 A1**
(43) Date de publication de la demande: **06.04.2011**
(21) Numéro de dépôt: 10184042.9
(22) Date de dépôt: 18.11.1999
(51) Int. Cl.: C12N 15/12, C07K 14/47, C07K 16/18, C12Q 1/68, A61K 38/17, A61K 39/395, A61P 25/16, A01K 67/027

(54) **Mutations du gène de la parkine, compositions, méthodes et utilisations**

(30) Priorité: 19.11.1998 FR 9814524; 12.03.1999 US 124239 P; 04.08.1999 FR 9910140
(62) Demande divisionnaire de: 09167384.8
(71) Demandeur: Aventis Pharma S.A., 92160 Antony (FR); INSERM (Institut National de la Santé et de la Recherche Medicale), 75654 Paris Cedex 13 (FR)
(72) Inventeur: Brice, Alexis, 75006 Paris (FR); Lucking, Christophe, 75015 Paris (FR); Abbas, Nacer, Eddine, 75015 Paris (FR); Denefle, Patrice, 75013 Paris (FR); Ricard, Sylvain, 75013 Paris (FR); Bouley, Sandrine, 75013 Paris (FR)
(74) Mandataire: Bouvet, Philippe

(57) **Abrégé**

La présente invention concerne des acides nucléiques codant pour des formes de la parkine humaine mutées, tronquées ou comportant des multiplications d'exons, et les protéines et anticorps correspondants. Elle concerne aussi des méthodes et kits pour l'identification de mutations du gène de la parkine, ainsi que pour l'étude de composés à visée thérapeutique.

## Description

La présente invention est relative au domaine de la génétique et, plus particulièrement, à l'identification de mutations du gène de la parkine. Elle concerne également des compositions et méthodes pour l'identification de ces mutations dans des échantillons, des formes de la parkine mutées, tronquées ou comportant des multiplications d'exons, et leurs utilisations à des fins de diagnostique, dépistage ou thérapeutique, par exemple.

La maladie de Parkinson est une affection neurodégénérative fréquente dont la prévalence est proche de 2% après l'âge de 65 ans [de Rijk et al., 1997]. Les signes cardinaux de la maladie sont rigidité, bradykinésie, tremblement de repos et bonne réactivité, au moins initiale à la lévodopa. Les troubles sont dus à une perte massive des neurones dopaminergiques de la substantia nigra. Les causes de la maladie restent inconnues mais l'intervention de facteurs de susceptibilité génétique est fortement suspectée [Wood, 1997]. De nombreuses formels familiales avec une transmission dominante ont été rapportées. Des mutations du gène codant pour la synucléine alpha, localisé en 4q21-q23, ont été décrites dans un petit nombre de familles avec un début précoce et une aggravation rapide [Polymeropoulos et al., 1997; Krüger et al., 1998]. Un second locus est situé en 2p13 [Gasser et al., 1998]. Un syndrome parkinsonien de transmission autosomique récessive (AR-JP) a été décrit au Japon [Yamamura et al., 1973; Ishikawa and Tsuji, 1996]. Il se manifeste avec les signes cardinaux de la maladie de Parkinson avec certaines particularités: i) début précoce, en règle avant I`âge de 40 ans; ii) présence d'une dystonie, souvent aux membres inférieurs; iii) fluctuations au cours de la journée; et iv) évolution lentement progressive mais toujours associée à des dyskinésies sous lévodopa. L'examen neuropathologique révèle une perte massive des neurones de la substantia nigra pars compacta mais sans corps de Lewy, stigmate histopathologique de la maladie de Parkinson idiopathique [Yamamura et al., 1973; Takahashi et al., 1994]. Une liaison génétique entre la maladie dans des familles japonaises et des marqueurs en 6q25.2-27 a été démontrée qui définit le locus PARK2 [Matsumine et al., 1997]. Deux équipes ont ensuite décrit des familles PARK2 hors du Japon, en particulier aux Etats Unis, en Europe et au Moyen Orient [Jones et al., 1998; Tassin et al., 1998]. Très récemment, Kitada et al [Kitada et al., 1998] ont mis en évidence des délétions des exons (3-7) ou de l'exan 4 d'un nouveau gène, appelé parkine, dans 4 familles japonaises.

La présente demande décrit maintenant la mise en évidence et la caractérisation, dans 77 familles et 102 cas isolés principalement européenne avec un syndrome parkinsonien de début précoce, de la présence de nouvelles altérations génétiques affectant le gène de la parkine. En outre, la présente demande montre que ces altérations génétiques sont présentes non seulement dans les syndromes parkinsoniens de début précoce, mais également dans les syndromes parkinsoniens plus tardifs ou atypiques. Ces nouvelles altérations offrent donc de nouveaux outils, à la fois pour le diagnostique et le traitement de la maladie de parkinson.

Un objet plus particulier de l'invention concerne un acide nucléique codant pour la parkine humaine, caractérisé en ce qu'il comporte une ou plusieurs altérations génétiques choisies parmi:
a) une délétion de un ou plusieurs exons, en combinaison ou non,
b) une multiplication (p.ex. duplication, triplication) d'exons,
c) une mutation ponctuelle,
d) une délétion de 1 ou plusieurs paires de bases contiguës entraînant un décalage du cadre de lecture, et
e) une insertion de 1 ou plusieurs paires de bases contiguës.

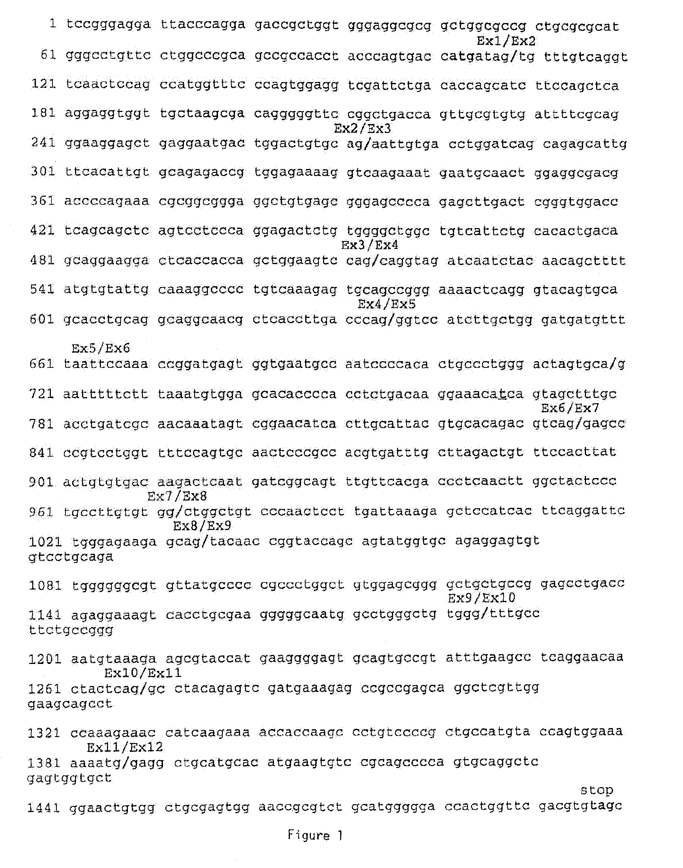

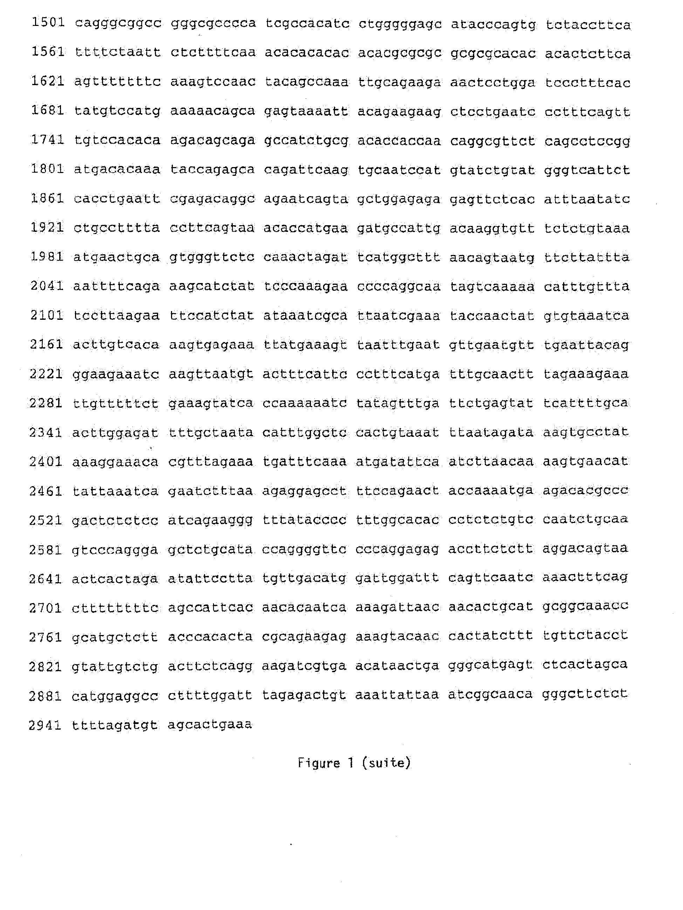

Le terme acide nucléique tel que défini dans la présente demande désigne les acides désoxyribonucléiques (ADN) et ribonucléiques (ARN). En outre, parmi les ADN, il peut s'agir d'ADN génomique (ADNg) ou complémentaire (ADNc). Les acides nucléiques de l'invention peuvent être d'origine naturelle ou synthétique. Il sont généralement préparés par les techniques classiques de biologie moléculaire, incluant le criblage de banques, 1a synthèse artificielle, la ligature, la restriction, etc. Les positions données dans la présente demande sont calculées par rapport à la séquence de la parkine humaine représentée sur la Figure 1 (SEQ ID No:1). Cette séquence représente la séquence de l'ADNc codant pour la parkine humaine. Par rapport à la séquence décrite par Kitada et al., elle comporte une modification au niveau du nucléotide 768 (C->T).

Les altérations génétiques définies ci-dessus a) à e) sont principalement des altérations exoniques, c'est-à-dire affectant la région codante du gène de la parkine humaine. Toutefois, il a été également mis en évidence des altérations introniques, c'est-à-dire dans la partie non codante du gène.

Le gène de la parkine humaine comprend 12 exons, dont les positions nucléotidiques sont données ci-après :

| | |
|---|---|
| Exon 1: nucléotides 1 à 108 | Exon 2: nucléotides 109 à 272 |
| Exon 3: nucléotides 273 à 513 | Exon 4: nucléotides 514 à 635 |
| Exon 5: nucléotides 636 à 719 | Exon 6: nucléotides 720 à 835 |
| Exon 7: nucléotides 836 à 972 | Exon 8: nucléotides 973 à 1034 |
| Exon 9: nucléotides 1035 à 1184 | Exon 10: nucléotides 1185 à 1268 |
| Exon 11: nucléotide 1269 à 1386 | Exon 12: nucléotides 1387 à 2960 |

Dans un premier mode de mise en oeuvre, l'invention concerne un acide nucléique codant pour la parkine, comportant des délétions de un ou plusieurs exons, notamment des combinaisons de délétions d'exons. Plus particulièrement, ces délétions affectent les exons 2 à 9 séparément ou en combinaison. Des exemples particuliers de ces délétions et combinaisons de délétions d'exons du gène de la parkine sont illustrés dans les tableaux 2 et 4. En outre, ces délétions peuvent être homozygotes c'est-à-dire affecter les deux chromosomes simultanément, ou hétérozygotes (incidence sur un chromosome seulement).

La demanderesse a en particulier mis en évidence la délétion hétérozygote de l'exon 2 ainsi que des combinaisons de délétions de cet exon avec d'autres exons dont notamment l'exon 3 et l'exon 4. Ainsi, neuf délétions ou combinaison de délétions ont été pour la première fois mises en évidence et notamment les délétions suivantes : exons 2, 2 + 3, 2 + 3 + 4, 3 - 6, 3 - 9, 6, 6 + 7, 7 + 8 + 9 et 8. Par ailleurs, ces délétions ou combinaisons de délétions peuvent être combinées entre elles en cas d'hétérozygotes composites.

La demanderesse a également mis en évidence un certain nombre de délétions homozygotes, seules ou combinées, telle que les délétions des exons 5 et 6.

Les positions particulières des délétions décrites ci-dessus peuvent être définies en se rapportant à la numérotation des nucléotides sur la figure 1.

Dans un autre mode particulier, l'invention concerne un acide nucléique codant pour la parkine, contenant une délétion de l'exon 3. Plus particulièrement, cette délétion affecte les nucléatides 273 à 513 sur la Figure 1.

Dans un autre mode particulier, l'invention concerne un acide nucléique codant pour la parkine, comprenant une délétion des exons 8 et 9. Plus particulièrement, cette délétion affecte les nucléotides 973 à 1184 sur la Figure 1.

Les conséquences de ces délétions ou combinaison de délétions restent souvent un décalage dans le cadre de lecture. Le tableau 4 résume les conséquences apparues en regard des délétions ou combinaisons de délétions relevées.

Dans un autre mode de mise en oeuvre particuliers, l'invention concerne un acide nucléique codant pour la parkine comprenant une multiplication d'exons, c'est-à-dire la répétition d'un ou plusieurs exons dans le gène. La présente demande montre pour la première fois : soit une duplication homozygote et hétérozygote comme cela est illustré dans les exemples par la duplication de l'exon 3, soit une duplication de type hétérozygote comme illustré par la duplication de l'exon 6, 7 ou 11. La présente demande montre également pour la première fois une triplication d'exons : soit une triplication homozygote, soit une triplication hétérozygote comme cela est illustré dans les exemples par la triplication de l'exon 2.

Préférentiellement, le terme multiplication d'exons indique la présence de 2 à 5 copies de ou des exons considérés, de préférence de 2 à 3 copies. Généralement, chaque copie d'exon est positionnée dans la séquence à côté de l'exon original.

Dans un autre mode particulier, l'invention concerne un acide nucléique codant pour la parkine, comprenant une mutation ponctuelle, c'est-à-dire le remplacement d'une paire de bases par une autre. La présente demande montre en effet l'existence de mutants ponctuels de la parkine et le caractère causal de certains d'entre eux dans l'apparition et le développement d'un syndrome parkinsonien. Plus particulièrement, la ou les mutations ponctuelles selon l'invention sont des mutations ponctuelles non-sens ou faux-sens ou bien entraînant un décalage du cadre de lecture,

Une mutation non-sens est une mutation introduisant dans la séquence un codon stop. Une telle mutation conduit à l'arrêt prématuré de la traduction, et donc à la synthèse d'une protéine tronquée. Une telle mutation est donc également désignée dans ce qui suit par le terme "tronquante". Plus préférentiellement, selon la présente invention, l'acide nucléique comprend une mutation non-sens localisée dans une région correspondant au domaine N- ou C-terminal de la Parkine humaine. La présente invention montre en effet que ce type de mutation se rencontre chez des patients plus fréquemment dans les régions terminale du gène (et donc de la protéine). Plus préférentiellement encore, la présente invention concerne un acide nucléique codant pour la parkine humaine, comprenant une mutation ponctuelle tronquante dans une région correspondant aux exons 2, 3, 11 ou 12. Il s'agit encore plus préférentiellement d'une mutation dans l'exon 12 du gène de la parkine, préférentiellement conduisant à l'inactivation du site de myristoylation (résidus 450-455 de la protéine). A titre illustratif, on peut citer la mutation ponctuelle G->A sur le nucléotide 1459, introduisant un codon stop à la place du résidu Trp453. Ce mutant code donc pour une protéine tronquée comprenant les 452 premiers acides aminés de la protéine sauvage. De manière surprenante, la demanderesse a montré que ce mutant Parkinel-452, qui est dépourvu seulement des 12 derniers résidus de la protéine sauvage, cause la maladie de Parkinson.

Selon un autre mode de réalisation, la présente invention concerne un acide nucléique codant pour la parkine humaine, comprenant une mutation ponctuelle tronquante dans l'exon 7. A titre illustratif, on peut citer la mutation ponctuelle T->A sur le nucléotide 905, introduisant un codon stop à la place du résidu Cystéine 268.

Un autre type de mutation ponctuelle selon l'invention est une mutation faux-sens. Une mutation faux-sens comprend le remplacement d'une paire de bases dans un codon, conduisant à un codon codant pour un acide aminé différent de l'acide aminé naturel, sans interruption de la séquence. Une telle mutation, isolée, conduit donc à une protéine ayant un nombre inchangé de résidus, mais dont l'un des résidus diffère de la protéine sauvage. La présente demande a maintenant montré que des mutants ponctuels faux-sens de la parkine existent chez des sujets atteints de syndromes parkinsoniens, et que ces mutants peuvent avoir un caractère causal. Plus préférentiellement, l'invention concerne un acide nucléique codant pour la parkine humaine, comprenant au moins une mutation ponctuelle faux-sens localisée notamment dans une région correspondant aux exons 4 à 12 et de manière préférée aux exons 4, 6, 7, 9, 11 et 12 .

Un premier type de mutations ponctuelles faux-sens plus particulières au sens de l'invention comprend les mutations qui entraînent un changement non-conservatif d'acide aminé dans la protéine codée. De telles mutations sont en effet plus spécifiquement associées au phénotype de la maladie de parkinson. On entend par changement non-conservatif le remplacement d'un acide aminé par un autre acide aminé ayant des propriétés structurales, physicochimiques et/ou biologiques différentes du premier. Ainsi, le changement d'un acide aminé basique par un acide aminé non-basique est dit non-conservatif. Ce type de changements comprend plus particulièrement le changement des acides aminés de l'une ou l'autre des catégories suivantes : acide, basique, neutre polaire, neutre non polaire. Des exemples particuliers de mutants de ce type selon l'invention sont notamment les acides nucléiques comprenant l'altération génétique suivante, seule ou en combinaison :
- une mutation A->T sur le nucléotide 584 (Lys161Asp) dans l'exon 4,
- une mutation A->T sur le nucléotide 734 (Lys211Asn) dans l'exon 6,
- une mutation C->T sur le nucléotide 867 (Arg256Cys) dans l'exon 7,
- une mutation C->T sur le nucléotide 924 (Arg275Trp) dans l'exon 7,
- une mutation G->A sur le nucléotide 939 (Asp280Asn) dans l'exon 7,
- une mutation G->A sur le nucléotide 1084 (Gly328Glu) dans l'exon 9,
- une mutation C->T sur le nucléotide 1101 (Arg334Cys) dans l'exon 9, et/ou
- une mutation G->A sur le nucléotide 1390 (Gly430Asp) dans l'exon 12.

Un deuxième type de mutations ponctuelles faux-sens plus particulières au sens de l'invention comprend les mutations conservatrices. De telles mutations couvrent tout remplacement d'un codon codant un acide aminé par un codon codant un acide aminé du même groupe. Par groupe d'acides aminés on entend les acides aminés dont les propriétés structurales, physico-chimiques et/ou biologiques sont très proches et sont définis selon les catégories suivantes acide, basique, neutre polaire, neutre non polaire. A titre d'exemple de mutation conservatrice, on peut citer de manière générale le remplacement du codon AAA (Lys) par le codon AGA (Arg), Lys et Arg faisant partie du même groupe d'acides aminés basiques. Un exemple particulier de ce type de mutation selon l'invention est notamment l'acide nucléique comprenant l'altération génétique suivante, seule ou en combinaison :
- une mutation T ->G sur le nucléotide 966 (Cys289Gly) dans l'exon 7.

Un troisième type de mutations ponctuelles faux-sens plus particulières au sens de l'invention comprend les mutations qui affectent un site potentiel de phosphorylation dans la protéine codée. La présente demande montre en effet que des mutants de ce type apparaissent chez certains sujets atteints de la maladie de parkinson, et constituent donc des événements qui participent au développement de la pathologie, en raison des fonctions biologiques connues des événements de phosphorylation. Des mutations particulières sont donc celles qui modifient un acide aminé phosphorylable en un résidu non-phosphorylable. A cet égard, les résidus susceptibles d'être phosphorylés sont par exemple les résidus sérine, thréonine et tyrosine.

Des exemples particuliers de mutants de ce type selon l'invention sont notamment les acides nucléiques comprenant l'altération génétique suivante, seule ou en combinaison:
- mutation C->A sur le nucléotide 1345 (Thr415Asn), exon 11.

Par ailleurs, comme indiqué ci-avant, la présente invention concerne également tout acide nucléique comprenant une délétion de une ou plusieurs paires de bases contiguës et entraînant un décalage du cadre de lecture (voir d). La présente demande démontre pour la première fois l'existence de mutants de délétion restreinte de la parkine, et leur implication dans l'apparition et le développement d'un syndrome parkinsonien. Plus préférentiellement, les délétions restreintes selon l'invention conduisent, en raison du décalage du cadre de lecture, à la synthèse de protéines (i) tronquées et (ii) dont la séquence C-terminale est différente de la protéine sauvage. Dans le cas de délétions introniques, le décalage du cadre de lecture peut conduire soit à la non reconnaissance de l'intron si la mutation a lieu à la jonction exon-intron et la production d'une protéine aberrante, soit à un pliage incorrect de l'intron empêchant ainsi l'excision-épissage de celui-ci, lorsque la mutation a lieu à l'intérieur de l'intron. Comme indiqué ci-avant, cette protéine (ou le domaine original) constitue un autre objet de la présente demande.

Plus préfér-entiellement, l'invention concerne les acides nucléiques comprenant une délétion de 1 à 10 paires de bases contiguës, de préférence de 1 à 5. Encore plus particulièrement, les délétions selon l'invention sont localisées dans une région de l'acide nucléique correspondant à l'intron 8 ou à l'exon 9 ou à une région terminale de la protéine, notamment dans les exons 2 ou 3, par exemple l'exon 2. A titre d'exempte spécifique, on peut citer un acide nucléique comportant les altérations suivantes, seules ou en combinaison:
- une délétion des nucléotides AG aux positions 202 et 203. Cette délétion introduit un changement du cadre de lecture débutant au niveau du résidu d'acide aminé 34 (Gln->Arg), et se terminant par un codon stop (37). Cette protéine est donc tronquée et comprend une région C-terminale de 4 acides aminés originale.
- une délétion du nucléotide A en position 255. Cette délétion introduit un changement du cadre de lecture débutant au niveau du résidu d'acide aminé 52 (Asn->Met), et se terminant par un codon stop (81). Cette protéine est donc tronquée et comprend une région C-terminale de 30 acides aminés originale.
- une délétion des cinq paires de bases TCTGC dans l'intron 8, en position - 21 -17 par rapport à l'exon 9 et pouvant entraîner une non reconnaissance du site d'épissage.
- une délétion de deux paires de bases dans l'exon 9 en position 1142-1143delGA qui change l'Arg348 en Glu. Cette délétion a pour conséquence l'introduction d'un changement du cadre de lecture créant ainsi un codon stop en position 368 dans l'exon 10.

Comme indiqué ci-avant, la présente invention concerne également un acide nucléique comprenant une ou plusieurs altérations génétiques, telles que notamment une insertion de une ou plusieurs paires de bases contiguës (voir e)). La présente demande démontre en effet pour la première fois l'existence de mutants d'insertion de la parkine, et leur implication dans l'apparition et le développement de la maladie de parkinson. Plus préférentiellement, l'insertion selon l'invention est telle qu'elle entraîne un décalage du cadre de lecture. De ce fait, l'insertion entraîne un changement des résidus situés en aval (du coté C-terininal) de la mutation. En outre, cette insertion conduit le plus généralement à la création d'un codon stop prématuré, et donc à la synthèse d'une protéine (i) tronquée et (ii) dont la séquence C-terminale est différente de la protéine sauvage. Comme indiqué plus loin, cette protéine (ou le domaine original) constitue un autre objet de la présente demande, et peut être utilisée comme outil diagnostique ou thérapeutique.

Plus préférentiellement, l'invention concerne les acides nucléiques comprenant une insertion de 1 à 5 paires de bases contiguës, de préférence de 1 ou 2. A titre d'exemple spécifique, on peut citer un acide nucléique comportant une insertion GT entre les nucléotides 321 et 322. Cette insertion introduit un changement du cadre de lecture débutant au niveau du résidu d'acide aminé 74 (Trp->Cys), et se terminant par un codon stop (81). Cette protéine est donc tronquée et comprend une région C-terminale de 8 acides aminés originale.

Bien entendu, les altérations génétiques a) à e) décrites ci-dessus peuvent être isolées ou combinées entre elles, telles que notamment une mutation faux-sens et une délétion ou encore deux mutations faux-sens cumulées. A titre d'exemples illustrant ce type de combinaison, on peut citer particulièrement la combinaison de modifications suivantes :
- une mutation faux-sens C->T en position 1101 (Arg334Cys) dans l'exon 9 avec une délétion de 5 paires de bases en position -21-17 par rapport à l'exon 9, dans l'intron 8
- une mutation faux-sens C->T en position 924 (Arg275Trp) dans l'exon 7 avec une mutation faux-sens G->A en position 1390 (Gly430Asp) dans l'exon 12.

L'invention a également pour objet un acide nucléique codant pour la parkine humaine, caractérisé en ce qu'il comporte la séquence présentée sur la figure 1 (SEQ ID No:1). Cette séquence comporte, par rapport à la séquence isolée par Kitada et al, une T en position 768, à la place d'une C, résultant, dans la protéine codée, en un acide aminé sérine en position 223, à la place d'une proline. Cet acide nucléique code pour la parkine sauvage rencontrée dans les populations européennes.

L'invention a également pour objet les variants polymorphiques de l'acide nucléique présenté sur la figure 1. La présente demande montre en effet que le gène de la parkine humaine présente un certain polymorphisme, et décrit plus particulièrement certains variants, présentant plus spécifiquement une des modifications de séquence suivantes:
- mutation G->A sur le nucléotide 601 de l'exon 4 (Ser167Asn)
- mutation G->C sur le nucléotide 1239 de l'exon 10 (Va1380Leu)
- mutation G->A sur le nucléotide 1281 de l'exon 11 (Asp394Asn).

L'invention concerne aussi tout vecteur comprenant un acide nucléique tel que défini ci-avant. Il peut s'agir d'un vecteur plasmidique, d'un cosmide, vecteur viral, épisome, chromosome artificiel, etc. Dans un mode particulier, un tel vecteur comprend aussi une région promotrice permettant l'expression dudit acide nucléique.

De tels vecteurs peuvent être utilisés pour produire en quantités importantes les acides nucléiques de l'invention, ou pour produire les polypeptides correspondants, dans un hôte cellulaire approprié (cellule procaryote, eucaryote, animale ou végétale, par exemple). Des hôtes cellulaires préférés sont notamment des cellules de bactérie (E. coli par exemple) ou des cellules de levure, ou encore des cellules mammifères, animales ou humaines.

A cet égard, l'invention concerne aussi toute cellule recombinante comprenant un acide nucléique ou un vecteur tels que définis ci-avant.

L'invention concerne aussi toute cellule mammifère, notamment humaine, comprenant un acide nucléique ou un vecteur tels que définis ci-avant, en remplacement du gène sauvage de la parkine.

Les cellules de l'invention peuvent être utilisées notamment pour l'étude des propriétés de la parkine, ainsi que comme modèles pour la recherche de composés susceptibles de compenser les altérations génétiques du gène de la parkine.

L'invention concerne en outre tout mammifère non-humain comprenant un acide nucléique tel que défini ci-avant dans ses cellules, Avantageusement, ces mammifères sont obtenus par "knock-in" des altérations identifiées ci-avant, par recombinaison homologue, ou également par "knock-out" du gène sauvage, substitué par la version altérée de l'invention.

De tels mammifères (rongeurs, canins, lapins, etc.) sont notamment utilisables pour l'étude des propriétés de la parkine et l'identification de composés à visée thérapeutique, par exemple.

L'invention concerne également tout polypeptide codé par un acide nucléique tel que décrit ci-avant. Ces polypeptides sont donc la parkine humaine, ses variants polymorphes, et des variants mutés et/ou tronqués et/ou comportant une multiplication d'exons, impliqués dans l'apparition et/ou le développement d'un syndrome parkinsonien. L'invention concerne en particulier les variants tronqués ou aberrants de la parkine tels que décrits ci-avant, ou une partie de ceux-ci correspondant à la séquence créée par le décalage de cadre de lecture. De tels polypeptides ou fragments, ou les acides nucléiques correspondants, sont utilisables pour identifier et/ou étudier des composés susceptibles de restaurer un phénotype normal à des cellules les exprimant. En particuliers, les acides nucléiques décrits ci-dessus peuvent être transférés dans des cellules hôtes appropriées, de préférence des cellules eucaryotes (mammifère, levure par exemple), pour être utilisés dans un test de screening de composés capables de contrecarrer leur activité, qu'il s'agisse de composés chimiques, biochimiques ou génétiques. De tels polypeptides ou fragments sont également utilisables à titre d'antigènes, pour la préparation d'anticorps spécifiques, utilisables pour la détection des variants. En particulier, les régions polypeptidiques spécifiques des formes tronquées (en particulier les extrémités) peuvent être utilisées pour la préparation d'anticorps, selon les techniques classiques de l'immunologie, lesquels anticorps constituent alors des outils de détection de la présence de ces formes dans des échantillons biologiques issus de sujets. De tels anticorps peuvent être polyclonaux ou monoclonaux (préparés par exemple par fusion de cellules de rate d'animaux immunisés avec les antigènes, avec des cellules de myelome, puis sélection de clones producteurs d'anticorps monoclonaux).

A cet égard, l'invention concerne également tout anticorps spécifique d'un polypeptide tel que décrit ci-avant, ou d'une région spécifique d'un tel polypeptide. Le terme "anticorps spécifique" désigne un anticorps ayant une affinité particulièrement supérieure pour l'antigène donné, par rapport à tout autre antigène.

L'invention concerne en outre toute composition comprenant un polypeptide ou un anticorps ou encore un vecteur ou une cellule hôte transformée par l'acide nucléique de l'invention, tels que décrits ci-avant. Ces compositions peuvent être conditionnées dans différents types de milieux (solutions salines, isotoniques, etc.) en présence de stabilisants ou de conservateurs, par exemple. Ces compositions peuvent être conservés au froid ou congelées, dans tout dispositif approprié (tube, boite, flacon, fiole, poche, etc.).

D'autre part, en plus des altérations génétiques exoniques décrites ci-avant, l'invention décrit également des altérations génétiques introniques du gène de la parkine, Ces altérations n'induisent pas de changement dans la séquence de la protéine codée, et constituent essentiellement des variants polymorphiques. Ces variants sont plus particulièrement décrits dans le Tableau 2.

L'une des applications de l'invention réside dans la détection de la présence de mutations dans le gène de la parkine., et leur corrélation avec la susceptibilité à la maladie de Parkinson, par exemple. A cet égard, l'invention concerne également différents outils (sondes, amorces, anticorps, etc.), utiles à la mise en oeuvre de telles méthodes de détection.

En particulier, l'invention concerne toute sonde ou oligonucléotide hybridant spécifiquement avec un acide nucléique tel que défini ci-avant.

Les sondes ou oligonucléotides spécifiques de l'invention comprennent généralement moins de 500 pb, plus préférentiellement moins de 300 pb. Typiquement un oligonucléotide spécifique de l'invention comprend de 5 à 100 pb, avantageusement de 5 à 50 pb. La longueur de l'oligonucléotide peut bien entendu être ajustée par l'homme du métier. Les sondes ou oligonucléotides de l'invention sont par ailleurs généralement marqués, de manière à permettre leur détection. Différents types de marquages connus de l'homme du métier peuvent être utilisés (marquage radioactif, fluorescent, enzymatique, chimique, terminal ou interne, etc.). Enfin, les sondes ou aligonucléotides de l'invention ont la capacité d'hybrider spécifiquement avec les acides nucléiques tels que définis ci-avant, c'est-à-dire avec les différentes formes altérées du gène de la parkine. L'hybridation est dite spécifique lorsque la sonde ou l'oligonucléotide hybrident, dans des conditions de stringence élevée, avec l'acide nucléique portant l'altération recherchée, et pas ou peu avec le même acide nucléique ne portant pas ladite altération. L'hybridation est donc dite spécifique lorsque le différentiel signal spécifique/bruit de fond est suffisamment grand pour pouvoir être détecté.

Les sondes ou oligonucléotides de l'invention sont donc généralement complémentaires d'une région au moins du gène de la parkine portant les altérations génétiques a) à d) décrites ci-avant. La complémentarité est généralement parfaite, de manière à assurer une meilleure sélectivité d'hybridation. Ces sondes ou oligonucléotides peuvent être synthétisés par toute technique connue de l'homme du métier, par exemple par clivage à partir des acides nucléiques décrits ci-avant, ou par synthèse artificielle, ou en combinant ces techniques. Ces sondes ou oligonucléotides sont utilisables pour l'identification, sur des échantillons biologiques, de la présence d'altérations génétiques du gène de la parkine.

L'invention concerne aussi un couple d'amorce pour l'amplification de tout ou partie d'un acide nucléique tel que décrit ci-avant, caractérisé en ce qu'il comprend:
- une première amorce complémentaire d'une région du gène de la parkine située en 5' d'une altération génétique, et
- une deuxième amorce complémentaire d'une région du gène de la parkine située en 3' de ladite altération génétique.

Les amorces de l'invention sont généralement complémentaires d'une région du gène de la parkine, et comprennent avantageusement moins de 30 pb.

L'invention concerne encore un procédé pour l'identification d'une altération génétique dans le gène de la parkine et en particulier la détection de délétion(s) et/ou multiplication (p.ex. duplication, triplication) d'exons à l'état homozygote et hétérozygote.

Ce procédé selon l'invention comprend :
i) la mise à disposition d'un échantillon comprenant le gène de la parkine,
ii) l'amplification (semi-quantitative) d'une partie au moins dudit gène, ladite partie comprenant une altération génétique telle que définie ci-avant, et
iii) la mise en évidence de la présence de l'altération génétique.

Avantageusement, dans le procédé de l'invention, l'échantillon est un échantillon de sang, tissu, plasma ou une culture de cellules, provenant d'un sujet, notamment d'un mammifère, en particulier d'un humain. Dans un mode préféré de mise en oeuvre, l'échantillon est prétraité de manière à rendre le gène de la parkine, ou une partie de celui-ci, accessible pour l'amplification. Ce prétraitement peut comprendre la lyse des cellules, un traitement enzymatique, une dénaturation, etc.

Avantageusement, l'amplification est réalisée au moyen d'un couple d'amorces tel que décrit plus haut ou celles décrites par Kitada et al. et incluses par référence.

A titre d'exemple particulier de couple d'amorces selon l'invention, on peut citer les amorces servant à la détection d'altérations dans l'exon 3 ou de mutations ponctuelles. Ainsi, la paire d'amorces suivante à été utilisée dans le cadre de l'invention :
For : 5'-(Hex)AATTGTGACCTGGATCAGC-3' (SEQ ID No:2) et
Rev : 5'-CTGGACTTCCAGCTGGTGGTGAG-3' (SEQ ID No:3)

Les amorces suivantes ont également été utilisées pour la mise en évidence des mutations ponctuelles suivantes:
- Asp280Asn :: 5'-GGCAGGGAGTAGCCAAGTTGAGGAT-3' (SEQ ID No:4) séquence sauvage G
- Arg334Cys :: 5'-AGCCCCGCTCCACAGCCAGCGC-3' (SEQ ID No:5) séquence sauvage G

La mise en évidence d'une altération génétique telle que décrite ci-dessus peut être réalisée par différentes techniques, et notamment par séquençage, PCR/restriction, ASO, PAGE, par PCR/multiplexe semi-quantitative, comme détaillé dans la partie expérimentale. Brièvement, cette méthode repose sur l'amplification par PCR semi-quantitative et en phase exponentielle d'ADN matrice. Suivant cette méthode la comparaison du taux relatif d'ADN matrice est suffisante pour mettre en évidence une perte de la quantité d'ADN (délétion d'exon(s)) ou au contraire une augmentation de la quantité d'ADN (multiplication d'exon(s)).

L'invention concerne en outre un kit pour la mise en oeuvre des procédés de l'invention, comprenant une sonde ou un oligonucléotide ou un couple d'amorces tels que décrits ci-avant. Les kits de l'invention comprennent avantageusement les réactifs appropriés à une réaction d'amplification et/ou d'hybridation, et, éventuellement, un support pour de telles réactions (filtres, membranes, chips, etc.).

La présente invention est particulièrement adaptée au diagnostique d'une susceptibilité à la maladie de parkinson, par la recherche d'une altération génétique telle que décrite plus haut dans le gène de la parkine.

La présente invention est également relative à l'utilisation des outils décrits ci-avant (acides nucléiques, sondes, polypeptides, anticorps, cellules, animaux) pour l'identification de composés capables de contrecarrer, au moins en partie, les effets d'une altération génétique du gène de la parkine, notamment dans un but thérapeutique. Ainsi, de tels composés peuvent être mis en évidence par mise en contact d'une composition (ou d'un produit) test en présence d'une cellule ou d'un animal tel que décrit ci-avant, et détection d'un effet phénotypique ou génotypique.

La méthode de l'invention peut notamment permettre l'identification de composés susceptibles d'être utilisés, seuls ou en combinaison avec d'autres produits ou traitements, pour le traitement (i.e., la diminution) de la maladie de Parkinson. De tels composés constituent un autre objet de la présente invention.

D'autres avantages et applications de la présente invention apparaîtront à la lecture des exemples qui suivent, qui doivent être considérés comme illustratifs et non limitatifs.

### EXEMPLES

### A - Légende des Figures

Figure 1: Séquence de l'ADNc codant pour la parkine humaine. Les jonctions entre les exons sont indiquées. Le codon initiateur (atg) et le codon stop (tag) sont en gras. Le changement C>T en position 768 est en gras et souligné.

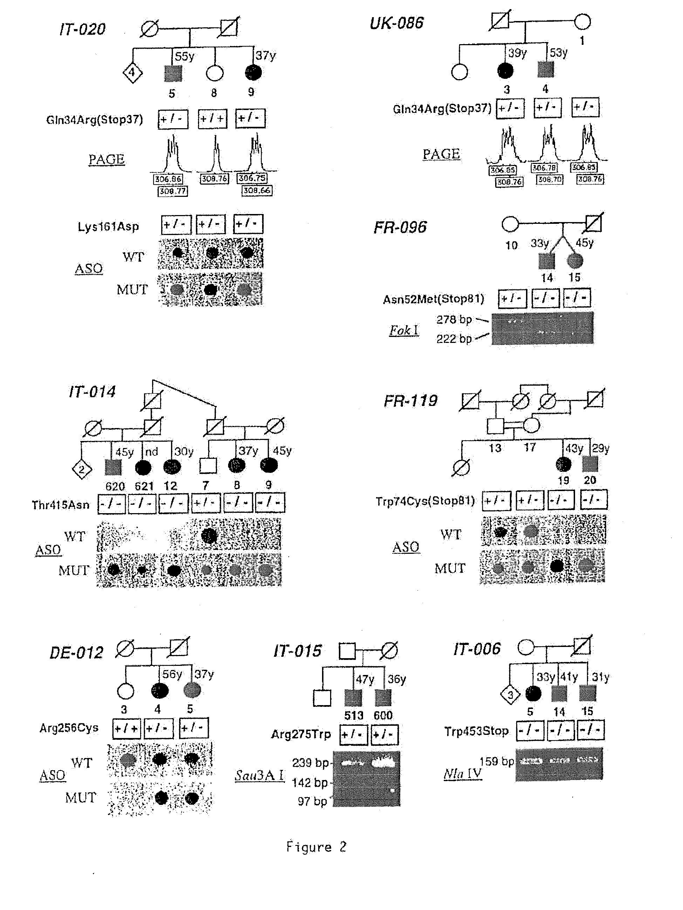

Figure 2: Familles présentant des mutations ponctuelles dans le gène de la parkine. La coségrégation complète de la mutation avec la maladie est représentée. Les carrés (homme) et ronds (femmes) noirs représentent les individus affectés avec l'âge d'apparition (en années) indiqué sous le symbole des patients. Les symboles barrés indiquent des patients décédés. Le nombre de frères et soeurs non affectés, non analysés est donné en losange. Pour chaque mutation (changement d'acide aminé), le génotype du membre de la famille est indiqué (+/+ homozygote sauvage, +/hétérozygote pour la mutation ; -/- homozygote pour la mutation). Sous chaque génotype, les résultats de détection sont donnés. PAGE : électrophoregranmes avec la taille de l'allèle en pb ; ASO : autoradiogrammes des allèles mutés et sauvages ; PCR/restriction : produits PCR après digestion avec les enzymes de restriction appropriées. La longueur des fragments en pb est donnée. Mut : muté ; nd : âge d'apparition non déterminé, puisque patient n'est pas conscient des symptômes.

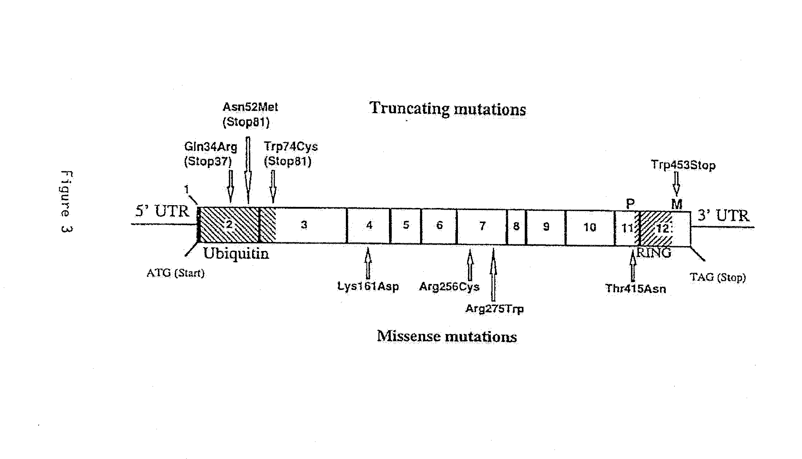

Figure 3 : Représentation et localisation des mutations ponctuelles du gène de la parkine. La séquence codante du gène, avec ses 12 exons, est représentée (barre). Les exons sont numérotés 1 à 12. 8 mutations ponctuelles causales sont positionnée selon leur effet sur la protéine (troncation vs faux-sens). Le domaine ubiquitine-like et le motif en anneau ("Ring Finger") sont hachurés. Pour les mutations Thr415Asn et Trp453Stop, les sites de phosphorylation (P) et de myristoylation (M) sont indiqués. UTR ; région non traduite.

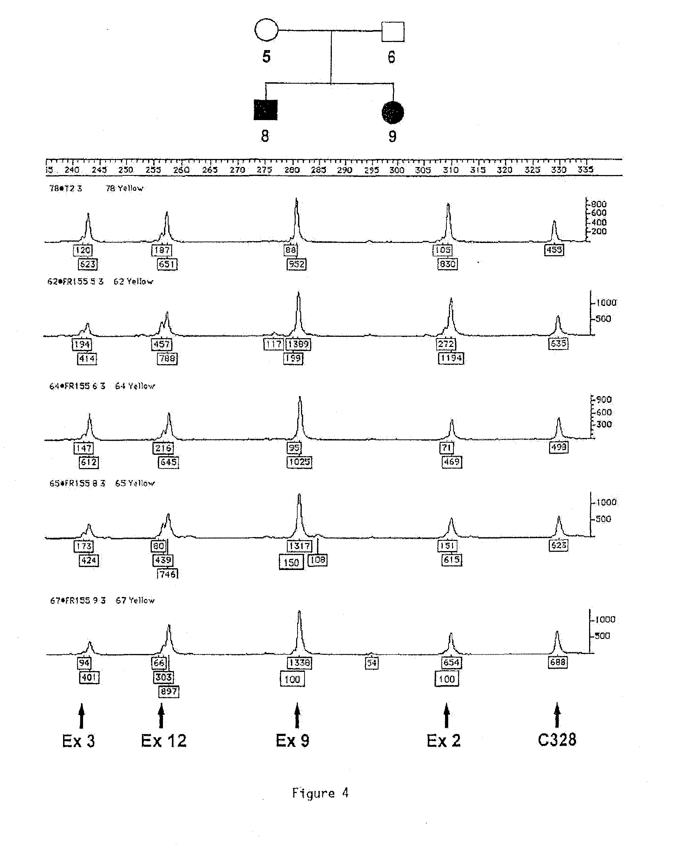

Figure 4: Résultats de la mise en évidence de délétions d'exons hétérozygotes dans une famille présentant le syndrome parkinsonien de début précoce (FPD-GRE-WAG-155) selon la méthode PCR/multiplexe semi-quantitative de l'invention. Les carrés (homme) et ronds (femmes) noirs représentent les individus affectés.

Les pics représentent les exons produits par PCR/nultiplexe semi-quantitative. Les chiffres entourés indiquent la hauteur des pics. La règle graduée au-dessus des éléctrophoregrammes indique la taille en paire de bases des produits de PCR.
Tableau 1 : Oligonucléotides utilisés pour la technique ASO. Les changements de nucléotide dans la séquence des oligonucléotides sont représentés en gras et soulignés. WT = type sauvage ; V = variant.
Tableau 2 : Récapitulatif des mutations mises en évidence. Les positions des nucléotides sont données selon la séquence de d'ADNc publiée dans la banque de données d'ADN japonaise (DDBJ; numéro d'accès AB009973) et sont illustrées dans la figure 1. PAGE = électrophorèse sur gel de polyacrylamide; ASO = technique de détection des mutations mettant en oeuvre un oligonucléotide spécifique d'un allèle.
Tableau 3 : Caractéristiques cliniques des patients en fonction du type d'altération génétique. Les patients de la famille IT-020 qui sont hétérozygotes composites pour une mutation faux sens et une mutation tronquante ne figurent pas dans le tableau. a: p<0,05 pour la comparaison entre les patients avec une délétion homozygote et les patients avec des mutations tronquantes.
Tableau 4 : Fréquence et conséquences des délétions/multiplications d'exons
   del = délétion, het = hétérozygote; hom = homozygote
Tableau 5 : Ratio des résultats obtenus dans la figure 4. La hauteur des pics est donnée pour chaque exon dans la partie gauche du tableau, les valeurs des doubles pics ayant été additionnées. La partie droite du tableau fourni le calcul des ratios des valeurs des pics. *Italique* = valeur normale; souligné = valeur pathologique comparée au contrôle. Dans la deuxième ligne du tableau, pour chaque cas, le ratio du témoin est divisé par le ratio du cas. Les valeurs pathologiques sont ou ≤0,625 ou ≥ 1,6 (=1/0,625). Des délétions d'exons ont été détectées pour les sujets FR 155 5 (exon 3), FR 155 6 (exon 2), FR 155 8 et FR 155 9 (exons 2 + 3). Pour ces deux derniers sujets atteints, la valeur du ratio exon 3/2 est normale étant donné que les deux exons ont été délétés de manière hétérozygote.

### B - Matériel et méthodes

### 1. Familles et patients

Dans une première série d'expériences, 38 familles ont été sélectionnées selon les critères suivants: syndrome parkinsonien réactif à la lévodopa, ii) âge de début au plus de 45 ans pour au moins un des membres atteints, et iii) transmission compatible avec une hérédité autosomique récessive.

Dans une autre série d'expériences, 77 familles ont été sélectionnées selon les critères suivants (comme indiqué dans Lücking et al, 1998; Abbas et al, 1999): i) présence d'un syndrome parkinsonien avec une bonne réponse à la levodopa (≥ 30% d'amélioration) chez au moins deux membres d'une fratrie (ou un seul s'il y a notion de consanguinité); ii) absence de critères d'exclusion tels que signe de Babinski, ophtalmoplégie, démence ou dysautonomie survenant avant deux ans d'évolution; iii) début ≤ 45 ans chez au moins un des atteints ; iv) hérédité compatible avec une transmission autosomique récessive (plusieurs patients dans une seule génération avec ou sans notion de consanguinité). Les familles étaient originaires de France (n=20), Italie (n=19), Grande-Bretagne (n=14), Pays-Bas (n=9), Allemagne (n=9), Liban (n=2), Algérie (n=1), Maroc (n=1), Portugal (n=1), Vietnam (n=1).

De plus, 102 cas isolés, sans consanguinité connue, ont été sélectionnés avec les même critères cliniques. Ils étaient originaires de France (n=31), Italie (n=23) et Grande-Bretagne (n=26), Allemagne (n=21)n Pays-Bas (n=1).

Tous les patients ont été évalués selon un protocole standardisé. Le consentement éclairé de tous les participants a été recueilli par écrit.

### 2. Analyse du gène de la parkine

L'ADN des 12 exons codants du gène de la parkine a été amplifié par PCR à partir de leucocytes du sang périphérique, pour chaque cas index, selon les conditions décrites dans Kitada et al. Brièvement, l'amplification a été réalisée sur 100 ng d'ADN, en présence de 350 µM de chaque dNTP, 350 µM de chaque amorce, et de la polymérase Taq. Les conditions d'amplification sont de 35 cycles à 94°C pendant 30sec., à 55-61 °C pendant 30sec., puis à 68°C pendant 30 sec. Pour les exons 4 et 7, seul le couple d'amorces introniques a été utilisé. La séquence des 12 exons a été réalisée sur les deux brins avec les amorces utilisées pour l'amplification par PCR, avec le kit de séquençage "Big Dye Terminator Cycle Sequencing Ready Reaction" (ABI PRISM) et analysée après électrophorèse sur séquenceur ABI 377 avec le logiciel "Sequence Analysis 3.0" (ABI PRISM).

La détection des mutations dans les échantillons et l'analyse d'une population de 45 individus contrôle a été effectuée par trois techniques, qui peuvent être utilisées seules ou en combinaison(s) : PCR/restriction avec l'enzyme de restriction appropriée ; technique ASO ("Allele Specific Oligonucleotide"), et électrophorèse sur gel de polyacrylamide ("PAGE"), comme illustré dans le Tableau 2. Plus particulièrement, ces techniques ont été mises en oeuvre comme décrit ci-après.

La technique ASO : Cette approche consiste à hybrider deux sondes oligonucléotidiques sur un échantillon amplifié (par exemple par PCR), la première spécifique de et recouvrant une altération génétique, la seconde spécifique de et recouvrant la région correspondante sauvage. Ainsi, en présence d'un gène muté, seule la première sonde permet l'hybridation avec le fragment d'ADN, alors qu'en présence d'un gène non-muté, seule la seconde sonde permet l'hybridation avec le fragment d'ADN. Dans le cas d'un gène hétérozygote, une hybridation est obtenue avec chacune des sondes. Cette technique peut également être réalisée de manière concomitante à l'amplification, en utilisant deux couples d'amorces, le premier comprenant une amorce spécifique de et recouvrant la région correspondante sauvage. Dans ce mode de réalisation, en présence d'un gène muté, seul le premier couple permet l'amplification d'un fragment d'ADN, alors qu'en présence d'un gène non-muté, seul le second couple d'amorce permet l'amplification d'un fragment d'ADN. Dans le cas d'un gène hétérozygote, un produit d'amplification est obtenu avec chacun des couples d'amorces.

Pour la mise en oeuvre de cette technique, 10 µl de produit PCR ont été dénaturés à 95°C pendant 5 min, déposés sur des membranes nylon Hybond N+ (Amersham), puis fixés au microonde à 600W pendant 2 min. Les amorces spécifiques (ou oligonucléotides) utilisées pour la détection (ou, le cas échéant, pour l'amplification) sont décrites dans les exemples (voir Tableau 1). Pour l'exon 3, les amorces exoniques Ex3iFor (avant) et Ex3iRev (arrière) ont été utilisées. La séquence de ces amorces est la suivante :
Ex3iFor :
   5'-AATTGTGACGTGGATGAGG-3' (SEQ ID NO : 6)
Ex3iRev :
   5'-CTGGACTTCCAGCTGGTGGTGAG-3' (SEQ ID NO :7)

Ces oligonucléotides (y compris les amorces, dans le cas d'une amplification simultanée), marqués au dCTP32 au moyen du kit Terminai Transferase (Boehringer Mannheim) ont été hybridés aux membranes à 44°C pendant la nuit dans un tampon consistant en 5 X SSC, 5 X Denhardts et 0,1% SDS. Les membranes ont ensuite été lavées deux fois pendant 30 min dans un milieu 2 X SSC à 59 °C et exposées à un film MP (Amersham) pendant 3-6 heures.

Technique de PCR/resiriction : Cette technique est basée sur l'utilisation d'enzymes de restriction dont le profil de digestion se trouve modifié du fait de l'altération génétique. Préférentiellement, cette technique met donc en oeuvre des enzymes de restriction dont le site est modifié (détruit ou créé) par l'altération génétique. Ainsi, selon le profil de digestion de l'acide nucléique (généralement du produit d'amplification), il est possible de distinguer la présence ou non de l'altération génétique recherchée. Bien entendu, cette technique est tout particulièrement adaptée à la recherche d'altérations génétiques caractérisées, entraînant une modification dans un site de coupure enzymatique. Pour sa mise en oeuvre, 15 µl de produit d'amplification est mis à digérer en présence de ou des enzymes de restriction appropriées, selon les recommandations du fabricant. Les enzymes particulières utilisées dans les exemples et la taille des fragments de restriction attendue sont donnés dans le Tableau 2.

Technique d'électrophorèse sur gel de polyacrylamide ("PAGE") : Cette technique permet de détecter la présence de mutations par mesure de la taille des produits d'amplification. Elle est donc tout particulièrement adaptée à la détection d'altérations génétiques de type insertion ou délétion. Pour sa mise en oeuvre, une amorce avant marquée (5-fluorescente, Hex) a été utilisée pour amplifier l'exon 2 du gène de la parkine. La présence de l'altération 202-203deIAG, résultant en un produit PCR plus court (306 vs 308 pb) a été établie par mesure de la taille du fragment amplifié en utilisant un séquenceur automatique ABI377 équipé des logiciels "Genescan 2.0.2" et "Genotyper 1.1.1" (ABI PRISM).

La numérotation des nucléotides utilisée dans la présente demande est donnée par référence à la séquence de l'ADNc qui figure dans la DNA Data Bank of Japan (DDBJ; accession number: AB009973). La séquence est représentée sur la figure 1. Cette séquence diffère de la séquence décrite par Kitada et al. au niveau du nucléotide 768. La séquence présentée sur la figure 1 correspond à la protéine sauvage rencontrée dans les populations européennes.

### 3. CR multiplexe semi-quantitative pour la détection de délétions/multiplications d'exons à l'état homozygote et hétérozygote

### a) Principes

La détection de délétions hétérogygotes ou des multiplications d'exons dans le gène de la *Parkine* ne peut pas être réalisée par PCR non quantitative. Ainsi, une PCR semi-quantitative qui compare le taux relatif d'ADN matrice est suffisante pour savoir s'il manque 50% de l'ADN matrice pour un ou plusieurs exons ou, au contraire dans le cas d'une multiplication hétérozygote ou homozygote, s'il y a p.ex. 50% (duplication hétérozygote), 100% (duplication homozygote ou triplication hétérozygote) ou 200% (triplication homozygote) d'ADN de trop pour un ou plusieurs exons. Pour réaliser cette comparaison, plusieurs exons d'un même individu sont amplifiés simultanément, dans une réaction de PCR (PCR multiplexe), les exons coamplifiés servant de standard interne de quantité. La PCR est réalisée avec des amorces fluorescentes, de telle sorte que la quantité de produit PCR peut être mesurée par la hauteur de pics sur un séquenceur automatique (ABI Prism 377), comme appliqué par exemple dans le LOH Assay (Loss of Heterozygosity) d'Applied Biosystems. La quantité de produit PCR (hauteur du pic) est directement liée à la quantité d'ADN matrice tant que la PCR est dans sa phase exponentielle qui signifie une absence de limitation par les substrats disponibles. Chaque PCR multiplexe, pour une combinaison donnée d'exons, produit une distribution typique de hauteur de pics pour un individu témoin et ainsi des ratios déterminés entre les différents pics.

Une délétion homozygote d'un exon sera démontrée par l'absence du pic correspondant. Si un exon est délété à l'état hétérozygote, le pic correspondant aura la moitié de sa hauteur normal, ce qui changera le rapport entre les exons délétés et non délétés par un facteur 2 par rapport à un témoin (comparant la valeur haute à la valeur basse; figure 4 et tableau 4 relatif à la figure 4). Pour les duplications d'exons, les rapports changent d'un facteur 1,5 pour les hétérozygotes et d'un facteur 2 pour les homozygotes (toujours en comparant la valeur haute à la valeur basse). Ainsi, les facteurs pour une triplication hétérozygote ou homozygote sont 2 ou 3, respectivement (toujours en comparant la valeur haute à la valeur basse). De façon à pouvoir détecter également une délétion ou une multiplication de l'ensemble du gène de la *Parkine,* un produit de PCR de 328 paires de bases (C328) d'un gène situé à distance (gène de la *Transthyrétine)* est amplifié et sert de standard externe dans une des PCR multiplexes. Le fait que seulement les rapports des hauteurs entre les pics soient comparées rend, en première approximation, la méthode indépendante de la quantité et de la qualité de I'ADN.

### b) Etablissement des conditions appropriées de PCR multiplexe

Au cours d'expériences préliminaires, il a été noté que les exons qui s'amplifient le mieux pouvaient influencer de façon négative l'amplification d'autres exons, pour lesquels l'efficacité était moins bonne. Ainsi, les exons dont l'efficacité d'amplification était comparable ont été regroupés. De plus, comme la taille du produit PCR peut influencer le rendement de l'amplification (les séquences courtes étant en règle mieux amplifiées que les longues), le regroupement des produits de PCR de taille comparable dans la réaction multiplexe a été effectué. Ainsi, trois combinaisons d'exons ont été choisies :
Comb 1: Ex 4o (261 bp) + 7o (239 bp) + 8 (206 bp) + 11 (303 bp),
Comb 2: Ex 5 (227 bp) + 6 (268 bp) + 8 (206 bp) + 10 (165 bp) et
Comb 3: Ex 2 (308 bp) + 3i (243 bp) + 9 (278 bp) + 12 (255 bp) + C328 (contrôle externe de 328 paires de base).

Les amorces utilisées sont celles décrites par Kitada et al (1998). Pour l'exon 3, une paire d'amorces exoniques a été utilisée:
For :5'-(Hex)AATTGTGACCTGGATCAGC-3' (SEQ ID NO : 8) et
Rev :5'-CTGGACTTCCAGCTGGTGGTGAG-3' (SEQ ID NO :9).
Les amorces pour C328 étaient :
TTREorHex : 5'-(Hex)ACGTTCCTGATAATGGGATC-3' (SEQ ID NO : 10) et
TTR328Rev: 5' -CCTCTCTCTACCAAGTGAGG-3' (SEQ ID NO : 11).

De façon à obtenir des pics de hauteurs comparables dans chaque PCR multiplexe et pour se situer dans la phase exponentielle pour chaque exon, les conditions de PCR ont été ajustées de façon permanente (en suivant en partie les recommandations de Henegariu et al (Henegariu et al, 1997). En particulier, les températures d'hybridation et d'extension ont été diminuées et la concentration de MgCl₂ et la durée de l'extension ont été augmentées. De plus, les concentrations d'amorces ont été ajustées à partir d'une concentration standard de 0,8µM, selon l'efficacité d'amplification (les concentrations d'amorces étaient diminuées pour les exons qui s'amplifiaient bien, et augmentées pour les autres).

Chaque combinaison d'exons a été testée pour vérifier que l'on situait dans la phase exponentielle et ce, dans deux PCR multiplexes en parallèle pour les 3 combinaisons d'amorces, sur un individu témoin avec 22, 23 et 24 cycles. Les hauteurs de pics ont été corrigées pour les variations de dépôts selon le marqueur de poids moléculaire interne (TAMRA 500 XL d'Applied Biosystems). Les hauteurs de pics corrigées étaient comparées au nombre de cycles, et représentent, sur une échelle logarithmique, une droite ascendante qui démontre que l'on situe dans la phase exponentielle pour les conditions suivantes:
5 minutes à 95°C pour un cycle,
30 secondes à 95°C, 45 secondes à 53°C et 2,5 minutes à 68°C pendant 23 cycles,
5 minutes à 68°C pendant un cycle.

La réaction était réalisée avec 40 ng d'ADN dans un volume de 25µl de solution de PCR, avec 3mM de MgCl₂, 0,2 mM dNTP et 1U Taq/25µl. La concentration de chaque primer était:
Ex 2 (0,8 µM), Ex 3 (0,4 µM), Ex 4 (1.0 µM), Ex 5 (0,6 µM), Ex 6 (1,4 µM), Ex 7 (o,44 µM), Ex 8 (dans comb 1: 1,0 µM et dans comb 2: 0,8 µM), Ex 9 (0,4 µM), Ex 10 (1,04 µM), Ex 11 (0,8 µM), Ex 12 (1,2 µM) et C328 (1,92 µM).

### c) Applications de la PCR multiplexe, contrôles internes et électrophorèse

En règle générale, les PCR multiplexes ont été effectuées au moins dans deux réactions parallèles pour chaque individu. Pour chaque série de patient, au moins un contrôle positif (avec une délétion hétérozygote d'exons connus) et un contrôle négatif (individu témoin) ont été traités en parallèle pour obtenir les valeurs normales et pathologiques pour chaque premix de réaction, de façon à éviter les résultats erronés dus à des différences possibles dans le premix (variation de pipetage). Deux témoins additionnels ont été ajoutés, qui ne contenaient pas d'ADN matrice. 1.5 à 2,5µl du produit de PCR ont été mélangés avec 4µl de tampon de chargement (incluant 0.3µl du marqueur de taille TAMRA 500 XL d'Applied Biosystems). 1,5 µl de ce mélange a été chargé sur un gel de polyacrylamide dénaturant à 4%, contenant 96 puits sur un séquenceur automatique ABI 377. Les gels sont analysés par les logiciels GeneScan 3.1 et Genotyper 1.1.1 (Applied Biosystems). Les hauteurs de pics sont mesurées comme indiqué dans Genotyper. Pour les doubles pics avec une paire de bases de différence (causées par le fait que la polymérase Taq ajoute inconstamment un A à chaque extrémité), les deux hauteurs de pics sont additionnées. Les rapports de chaque combinaison de pics sont calculés pour chaque réaction, utilisant le logiciel Excel 5.0 (tableau 5) et les valeurs moyennes sont calculées pour deux réactions.

### d) Interprétation

Pour les délétions, les résultats sont interprétés comme pathologiques si la différence de rapport était un facteur d'au moins 1,6 ou ≤0,625 (=1/1,6) dans tous les rapports respectifs entre le témoin et le cas (ratio du sujet/ ratio du control - tableau 5 relatif à la figure 4). Lorsque des différences de rapports entre les réactions parallèles sont discordantes (par exemple, à cause d'une amplification faible dans l'une des PCR), les rapports obtenus avec une amplification satisfaisante sont pris en compte à condition qu'ils soient normaux.

Pour les duplications, un changement des rapports d'un facteur 1,30-1,65 ou >1,75 est interprété comme une duplication hétérozygote ou homozygote, respectivement (en comparant la valeur haute à la valeur basse).

Pour une triplication, un changement des rapports d'un facteur 1,6-2,4 ou >2,6 est interprété comme une triplication hétérozygote ou homozygote, respectivement (en comparant la valeur haute à la valeur basse).

Cependant, comme les conditions ont été ajustées en permanence pendant le développement de la méthode, quelques-uns des résultats ont été obtenus dans des conditions légèrement différentes. Ces résultats sont pris en compte lorsqu'ils sont clairement normaux ou pathologiques et reproductibles. Dans les situations ambiguës, l'expérience était répétée dans des conditions adaptées.

### 4. Analyse de la coségrégation et d'une population témoin

### a) Mutations ponctuelles

Les variants de séquence de la *Parkine* ont été testés pour leur coségrégation dans les familles (selon la disponibilité d'autres échantillons) et pour leur présence dans une population de témoins sans syndrome parkinsonien (61 à 73 individus). A cause du caractère certainement pathogène de la mutation 1142-1143delGA, des témoins n'étaient pas testés pour cette mutation. Les techniques utilisées sont PCR et digestion avec l'enzyme de restriction appropriée ou électrophorèse en gel de polyacrylamide (PAGE = polyacrylamide gel electrophoresis) (voir tableau 2). Quand le variant ne provoquait pas de changement de site de restriction par lui-même, un site était artificiellement créé à l'aide d'une amorce avec un mismatch. Les amorces étaient conçues de façon à introduire le changement d'une base à proximité de la position du variant de séquence, de façon à créer un site de restriction qui inclut ce variant. Les amorces sont indiqués dans le tableau ci-dessous.

Amorces modifiécs (non complémentaires de la séquence sauvage pour une base) pour PCR :

| Mutation | Enzyme de restriction | Primer F | Primer R |
|---|---|---|---|
| Asp280Asn | Alw I | Ex 7o For | 5'-GGCAGGGAGTAGGCAAGTTGAGGAT-3' |
| | | | (SEQ ID NO: 12) |
| | | | séquence sauvage G |
| Arg334Cys | BstU I | Ex 9 For | 5'-AGCCCCGCTCCACAGCCAGCGC-3' |
| | | | (SEQ ID NO: 13) |
| | | | séquence sauvage G |

Le changement de paire de bases introduit est souligné par comparaison à la séquence sauvage.

### b) Délétions ou multiplication d'exons homozygotes et hétérozygotes

La coségrégation d'une délétion ou d'une multiplication d'exons dans les familles était analysée à l'aide des méthodes précédemment décrites. Une population contrôle n'a pas été testée en raison du caractère très probablement pathogène des mutations, qui entraîne une délétion interne de la protéine, avec ou sans décalage du cadre de lecture.

### 5. Analyse des liaisons génétiques

Pour tester la liaison au locus PARK2, quatre marqueurs de type microsatellite, situés à proximité du locus, étaient testés (D6S1579, D6S411, D6S1550 et D6S305) comme décrits par Tassin et al (1998).

### C - Résultats

*a) Dans une première série d'expériences, l'analyse du gène de la parkine a été réalisée chez le cas index de 38 familles avec AR-JP qui comportent 87 patients.*

### 1. Détection de délétions d'exons

L'amplification des exons a révélé la présence d'une délétion à l'état homozygote dans trois familles: délétion de l'exon 3 dans une famille française (SAL-024) et une portugaise (SAL-711), et des exons 8 et 9 dans une famille algérienne (DEL-001). Ces délétions se transmettent avec la maladie car elles sont détectées dans chaque famille à l'état homozygote chez tous patients mais pas chez les apparentés sains prélevés (figure 2).

### 2. Détection de mutations ponctuelles

L'analyse de séquence dans les famines sans délétion homozygote a révélé la présence de 16 variants de la séquence nucléique: 12 dans les exons et 4 dans les introns (tableaux 2 et 3, figure 3). Trois variants entrainent un décalage du cadre de lecture et la synthèse d'une protéine tronquée. Il s'agit des mutations 202-203delAG (Gln34Arg(Stop37)) et 255delA (Asn52Met(Stop81)) dans l'exon 2 et 321-322insGT (Trp74Cys(Stop81)) dans l'exon 3 trouvées respectivement dans les familles IT-020 et UK-086, TOU-096, LYO-119. Ces mutations, à l'exception de 202-203delAG sont à l'état homozygote. Une mutation non sens 1459G>A (Trp453Stop) dans l'exon 12 est présente à l'état homozygote dans la famille IT-006. Huit des variants sont de type faux sens. Dans l'exon 4, 584A>T (Lys161Asp) et 601G>A (Ser167Asn) sont à l'état hétérozygote chez les patients des familles IT-020 et SAL-730, respectivement. Dans l'exon 7, les variants 867C>T (Arg256Cys) et 924C>T (Arg275Trp) sont trouvés à l'état hétérozygote dans les familles DE-012 et IT-015, respectivement. Dans l'exon 10, le variant 1239G>C (Val380Leu) est trouvé à l'état hétérozygote et homozygote dans 11 familles (IT-014, IT-020, IT-058, SAL-017, GRE-029, SAL-038, TOU-096, SAL-431,UK-006, UK-086, DE-022). Dans l'exon 11, le variant 1281G>A (Asp394Asn) est détecté à l'état hétérozygote dans la famille UK-046 et 1345C>A (Thr415Asn) à l'état homozygote dans la famille IT-014. Enfin, le variant 768C>T (Pro223Ser) n'est pas pathogène, car il est détecté à l'état homozygote chez tous les individus séquencés suggèrant qu'il s'agit d'une erreur typographique dans la séquence de la parkine [Kitada et al., 1998]. La recherche de ces variants dans la population témoin révèle que trois d'entre eux représentent des polymorphismes (tableau 2) : Ser167Asn, Val380Leu et Asp394Asn. Les autres variants constituent très probablement des mutations causales car ils entrainent la synthèse de parkine tronquée ou des substitutions non conservatives ou des substitutions portant sur un des acides aminés susceptible d'être phosphorylés. De plus, ils ségrègent avec la maladie dans les familles et ne sont pas détectés dans 90 chromosomes de témoins.

Les variants identifiés dans les introns 2, 3, 6 et 7 (IVS2+25T>C (272+25T>C), IVS3-20C>T (514-20C>T), IVS6+19T>C (835+19T>C) et IVS7-35A>G (973-35A>G)) constituent des polymorphismes (tableau 2). Ils ne sont pas localisés à proximité des sites d'épissage et sont détectés dans les chromosomes de témoins.

### 3. Domaines fonctionnels de la Parkine

Une étude des domaines fonctionnels de la parkine a été entreprise par analyse et comparaison de séquences. Cette étude montre que le changement conservatif d'acide aminé Thr415Asn est localisé dans la séquence consensus d'une protéine kinase cAMP- et cGMP-dépendante (KKTT) et dans le site de phosphorylation d'une protéine kinase C (TTK). Cette étude montre en outre que la mutation non-sens Trp453Stop est localisée dans un site N-terminal de myristoylation (GCEWNR).

### 4. Corrélations phénotype génotype

Les délétions homozygotes et les mutations ponctuelles ont été détectées dans 12 famines qui comportent 26 patients. L'âge moyen de début est de 36,7 ans avec des extrèmes de 7 à 56 ans (tableau 3). La comparaison entre les familles selon les conséquences fonctionnelles des mutations (délétion homozygote, mutation tronquante et mutation faux sens) ne révèle pas de différences significative de l'âge de début, de la sévérité ou de la fréquence des signes associés, sauf pour le tremblement qui est significativement moins fréquent dans les familles avec une délétion homozygote, comparé aux familles avec des mutations tronquantes (tableau 3).

### b) Détection de nouvelles mutations ponctuelles

Huit nouvelles mutations ponctuelles dans des exons ont été identifiées, dont six sont des mutations faux-sens, une tronquantes et une non-sens: 734A>T (Lys211Asn) dans l'exon 6, 905T>A (Cys268Stop), 939G>A (Asp280Asn) et 966T>G (Cys289Gly) dans l'exon 7, 1084G>A (Gly328Glu), 1142-1143delGA et, 1101C>T (Arg334Cys) dans l'exon 9 et 1390G>A (Gly430Asp) dans l'exon 12. Cinq des mutations faux-sens conduisent à des changements d'acides aminés non conservatifs et une à un changement conservatif (Cys289Gly). De plus, une délétion de cinq paires de bases dans l'intron 8, localisée aux positions -21 à -17 par rapport à l'exon 9 a été mise en évidence. Tous ces variants de séquence n'ont pas été détectés chez 61 à 73 individus témoins (la mutation 1142-1143delGA n'étant pas testée) et ne représentent donc pas des polymorphismes. Les résultats sont détaillés dans le tableau 2.

### c) Détection de nouvelles délétions homozygotes d'exons

Des délétions homozygotes d'exons ont été détectées dans 3 familles en plus des délétions rapportées précédemment par Hattori et al (1998a) et Lücking et al (1998) pour l'exon 3 et par Hattori et al (1998a) pour les exons 3+4. Ces déplétions concernent les exons 3 (FDP-ANG-GEO-141), 3+4 (IT-064) et 5+6 (SPD-LIB-HAG-076). Les conséquences des délétions d'exons sur le cadre de lecture et leur fréquence relative dans l'échantillon sont indiquée dans le tableau 4.

### d) Détection des duplications/triplications homozygotes et hétérozygotes

Cinq nouveaux types de duplications d'exons ont été détectés: une duplication de l'exon 3 à l'état homozygote (SPD-NIC-AIT-091) et une duplication de l'exon 3 à l'état hétérozygote (SAL 399 213). En plus, des duplications hétérozygotes de l'exon 6 (FPD-LIL-CHA-171), de l'exon 7(DE 4001) et de l'exon 11 (SAL 399 213) ont été détéctées.Deux types de triplication ont été détectés: une triplication de l'exon 2 à l'état homozygote (RM 347) et a l'état hétérozygote (RM 330).

### e) Détection de nouvelles délétions hétérozygotes

Treize différentes combinaisons de délétions hétérozygotes d'exons ont été mises en évidence dans 21 familles. Les délétions suivantes ont été observées: exons 2, 2+3, 2+3+4, 3, 3+4, 3-6, 3-9, 4, 5, 6, 6+7, 7+8+9 et 8. Les délétions d'exons 2, 2+3, 2+3+4, 3-6, 3-9, 6, 6+7, 7+8+9 et 8 sont nouvelles.

Pour deux familles (Sal-Hab-436 et UK 12416), il n'a pas pu être établi avec certitude si les mutations hétérozygotes des exons 2+3 ou 6-7, respectivement, étaient situées sur le même chromosome ou s'il s'agissait des cas hétérozygotes composites à cause de l'absence d'ADN pour d'autres membres de ces familles. Les conséquences des délétions et des multiplication d'exons décrites sur le cadre de lecture et leur fréquence relative dans notre échantillon sont indiquées dans le tableau 4.

### f) Mutations ponctuelles récurrentes

Cinq mutations ponctuelles ont été mises en évidence dans plus d'une famille. Ces mutations sont 202-203delAG (à l'état hétérozygote ou homozygote dans 5 familles), 255delA (à l'état homozygote ou hétérozygote dans 6 familles), Lys211Asn (à l'état hétérozygote dans 2 familles), et Arg275Trp (à l'état hétérozygote dans 5 familles).

### g) Fréquences des différents types de mutations et des hétérozygotes composites

Parmi les familles avec des mutations de la *Parkine,* des délétions homozygotes d'exons ont été détectées dans 8 familles, des mutations ponctuelles à l'état homozygote dans 10 familles, une duplication d'exon homozygote dans une famille et une triplication d'exon dans une famille. Les patients de 21 familles étaient hétérozygotes composites pour deux mutations différentes (3 fois pour les mutations ponctuelles différentes, 6 fois pour une mutation ponctuelle et une délétion d'exon, deux fois pour une mutation ponctuelle et une duplication, une fois pour une triplication et une délétion d'exon une fois pour deux duplications d'exon différentes et 6 fois pour deux délétions différentes d'exons; voir exemple figure 4). Dans deux cas, il n'était pas possible de déterminer s'il s'agissait d'hétérozygotes aux délétions de plusieurs exons adjacents composites, et dans 13 cas, seule une mutation à l'état hétérozygote (6mutations ponctuelles et 7 mutations d'exons) était détectée.

### D) Discussion

La présente invention se rapporte à des variants du gène de la *Parkine,* leur utilisation diagnostique et/ou thérapeutique, ainsi que des techniques pour la détection d'altérations (notamment de délétions d'exons hétérozygotes et de multiplications d'exons) du gène de la *Parkine.*

La mise en évidence de différentes altérations génétiques (notamment de délétions homozygotes, de mutations ponctuelles, d'insertions et de multiplications d'exons) causales démontre que les anomalies du gène de la parkine constituent une cause fréquente d'AR-JP.

### 1. Première étude sur 38 familles européennes

Une première étude a permis de mettre en évidence l'existence de délétions, mutations et insertions dans le gène de la parkine

Le rôle pathogène des délétions homozygotes parait facile a établir. Dans les 2 mutations décrites, délétions de l'exon 3 et des exons 8-9, la perte de l'exon s'accompagne d'un décalage du cadre de lecture conduisant à l'apparition d'un codon de terminaison prématuré. En l'absence d'épissage alternatif, il en résultera un protéine tronquée.

Huit des variants exoniques constituent des mutations causales. Premièrement, ces mutations ségrègent avec la maladie dans les familles. Deuxièmement, ces variants ne sont pas détectés par ASO, PAGE ou PCR/restriction dans 90 chromosomes de témoins. Troisièmement, les conséquences fonctionnelles des mutations apparaissent délétères. 11 est facile de concevoir que les 4 mutations ponctuelles tronquantes (Gln34Arg(Stop37), Asn52Met(Stop81), Trp74Cys(Slop81), Trp453Stop), détectées à l'état homozygotes chez les patients de 3 des 5 familles, vont causer une perte de fonction de la parkine en accord avec la transmission autosomique récessive de la maladie. Trois des quatre mutations faux sens entraînent des changements non conservatifs d'acides aminés. L'un d'entre eux (Lys161Asp) est associé à une mutation tronquante sur l'autre allèle qui renforce l'hypothèse d'un rôle pathogène. Une mutation faux sens est conservative (Thr415Asn), mais affecte un site potentiel de phosphorylation. Trois des mutations faux sens sont présents à l'état hétérozygote chez des patients dont l'autre mutation n'a pas été caractérisée. Il est probable qu'il s'agit de délétions d'un ou plusieurs exons à l'état hétérozygote qui ne peuvent être visualisées avec les techniques utilisées pour cette étude.

Les anomalies du gène de la parkine mises en évidence sont variées et il n'existe pas de hot spot de mutations. Il faut noter que les mutations ponctuelles tronquantes correspondent préférentiellement aux régions N- et C-tertninales de la parkine (comprenant en particulier les motifs ubiquitine-like et en anneau "RING-fmger", respectivement) alors que les mutations de type faux sens affectent la région centrale. Seules deux des 11 mutations décrites dans cette première étude sont rencontrées dans plusieurs familles. La délétion homozygote de l'exon 3 est détectée dans les familles française SAL-024 et portugaise SAL-711. La mutation avec décalage du cadre de lecture 202-203delAG (Gln34Arg(Stop37)) est visualisée à l'état hétérozygote dans les familles italiennes IT-020 et anglaise UK-086. L'origine différente des familles est en faveur de l'hypothèse de la survenue indépendante de ces mutations.

Les mutations décrites affectent des familles provenant de 6 pays: Algérie, Allemagne, Angleterre, France, Italie et Portugal. L'étude du phénotype dans les familles avec une mutation montre que le spectre clinique associé aux anomalies de la parkine est plus étendu que dans les familles japonaises [Kitada et al., 1998]. Ces résultats confirment les observations faites dans des familles Européennes et d'Afrique du Nord étudiées par liaisons génétiques [Tassin et al,, 1998]. L'age de début est supérieur à 50 ans chez plusieurs patients, allant jusqu'à 56 ans. Certains signes cliniques tels que la dystonie ou les signes pyramidaux aux membres inférieurs ne sont pas toujours présents chez les porteurs de mutation même après des durées d'évolution de plusieurs décennies. Globalement le phénotype reste très similaire entre les groupes de patients classés selon les conséquences fonctionnelles des mutations. Cependant, la présence d'épisodes de dystonie douloureuse semble rencontrée exclusivement chez les patients porteurs de délétions homozygotes. L'absence de différence significative pour l'âge de début, la sévérité et la fréquence des signes associés entre les mutations ponctuelles tronquantes et les faux sens suggère que les acides aminés modifiés dans ces dernières jouent un rôle important dans la physiologie de la parkine.

En conclusion, cette première étude souligne la fréquence des mutations du gène de la parkine dans les syndromes parkinsoniens familiaux de début précoce en Europe. Des anomalies de ce gène sont aussi à l'origine de syndromes parkinsoniens plus tardifs ou atypiques. Il reste à déterminer le rôle de mutations de la parkine ou de ses polymorphismes dans les cas isolés. Les mutations détectées sont très diverses tant par leur nature que par leur localisation. L'étude de leur localisation dans la parkine suggère que de nombreuses régions de la protéine concourent à sa fonction, encore inconnue.

### 2. Méthode de détection des délétions d'exons et des multiplications d'exons

Pour la première fois, la détection de délétions d'exons à l'état hétérozygote et de multiplications d'exons (p.ex, duplication, triplications) à l'état homozygote et hétérozygote dans le gène de la *Parkine* est décrite. Cet aspect est intéressant car les délétions d'exons sont relativement fréquentes (voir plus loin). Comme méthode de détection, un protocole de PCR multiplex semi-quantitative a été choisi et mis au point. Cette méthode avait précédemment été validée pour le dosage génique, par exemple pour la détection de délétions du gène PMP22 (Poropat et Nicholson, 1998), à condition que l'amplification par PCR soit dans la phase exponentielle. Dans toutes ces expériences, le choix de témoins co-amplifiés qui servent de standard pour la quantification est crucial (Prior, 1998). Dans l'expérience, les exons non délétés servent de témoins internes dans l'amplification par PCR multiplexe chez le même individu. Les combinaisons de 4 ou 5 exons ont été choisies de façon à ne pas contenir plus de 2 exons adjacents, car de tels exons ne peuvent pas servir de contrôles dans le cas d'une délétion des deux. L'exon d'un gène différent (*Transthyrétine*) a été co-amplifié dans l'une des trois combinaisons pour identifier des délétions hétérozygotes de l'ensemble du gène *Parkine.* Ce contrôle externe était indirectement représenté dans les deux autres combinaisons, qui incluent des exons de par et autre de l'exon 9; ce dernier étant testé avec la *Transthyrétine.*

Les résultats obtenus par cette méthode étaient très reproductibles et les résultats anormaux montrent des différences dans les rapports d'un facteur, qui correspond à celui attendu en théorie. Ces résultats montrent qu'il s'agit d'une méthode simple et validée pour un criblage rapide. De plus, des petites délétions ou insertions dans le produit PCR, qui sont relativement fréquentes (voir ci-dessous) peuvent être détectées simultanément par cette méthode.

### 3. Délétions d'exons et multiplications d'exons

Il a été possible d'identifier quatre duplications d'exons et une triplication d'exons jamais décrites dans le gène de la Parkine auparavant, mais dont la fréquence relative est faible. De plus, 10 combinaisons de délétions d'exons nouvelles ont été identifiées avec, pour la première fois, la démonstration des délétions qui emportent l'exon 2. La fréquence relative des mutations ponctuelles et des délétions d'exons a été estimée à environ 50%. Ainsi, les délétions d'exons (hétérozygotes ou homozygotes) peuvent représenter jusqu'à 50% des mutations de la *Parkine,* soulignant l'intérêt de la technique décrite ici. De fait, cette technique a permis de détecter des mutations dans 26 des 53 familles. Ainsi, dans l'échantillon étudié les mutations ponctuelles et les délétions dans le gène de la *Parkine* ont la même fréquence, alors que les délétions d'exons sont majoritaires dans la population japonaise (Hattori et al, 1998). Les conséquences fonctionnelles des délétions ou des multiplications d'exons décrites (décalage du cadre de lecture ou délétion/multiplication en phase) ont été déduites des séquences d'ADNc publiées de la *Parkine* (Kitada et al, 1998) et sont spéculatives, car l'absence de produit de PCR n'indique pas qu'il y a forcément délétion de l'exon dans sa totalité (voir ci-dessus). Cependant, le rôle pathologique des modifications détectées est très probable, car elles se transmettent avec la maladie dans les familles qui ont pu être testées, elles sont associées à des mutation ponctuelles chez des hétérozygotes composites et les délétions sont identifiées avec une fréquence similaire à celle des mutations ponctuelles. De même, dans les cas isolés, la fréquence des délétions hétérozygotes et des mutations ponctuelles est similaire. Dans le cas de l'exon 3, des amorces exoniques ont été utilisées, qui démontrent l'altération de cet exon lorsqu'il n'y a pas de produit de PCR. En plus, dans une partie des cas, plusieurs exons juxtaposés étaient délétés simultanément, ce qui est un argument pour une grande délétion génomique.

Des délétions ou des multiplications hétérozygotes de l'ensemble du gène de la *Parkine* n'ont pas été observées. Il s'agit probablement d'un événement rare compte tenu de la très grande taille (environ 500 kb) de ce gène (Kitada et al, 1998).

Les délétions d'exons observées touchent fréquemment les exons 3 à 5. Cette observation est confirmée dans les familles européennes. De plus, il est démontré que l'exon 2 seul ou associé à d'autres, est aussi fréquemment impliqué dans les familles européennes (tableau 2).

### 4. Nouvelles mutations ponctuelles

L'identification de 8 nouvelles mutations ponctuelles (6 de type faux-sens, 1 tronquantes et 1 de type non-sens) augmente la diversité des mutations ponctuelles du gène de la *Parkine.* Les mutations décrites sont pathogènes, comme a montré la ségrégation avec la maladie, et ne sont pas détectées chez 122 à 147 chromosomes de témoins (la mutation 1142-1143delGA pas inclue), Même si le changement Cys289Gly est conservatif, ce changement d'acide aminé peut avoir des conséquences délétères importantes, si la cystéine en position 289 est impliquée dans un pont disulfure, important pour la fonction de la protéine.

De façon intéressante, 2 patients de la famille UK-040 présentent 3 mutations différentes (voir tableau 5) : une mutation faux-sens Arg334Cys dans l'exon 9 à l'état homozygote, une délétion homozygote de 5 paires de bases en position -17 à -21 de l'intron 8, et une mutation faux-sens non conservative Asp280Asn à l'état hétérozygote. On peut suspecter que la mutation Arg334Cys à l'état homozygote est causale, mais la délétion de cinq paires de base à l'état homozygote, à proximité du site accepteur épissage de l'exon 9, pourrait aussi avoir des conséquences fonctionnelles.

Cinq mutations ponctuelles sont présente dans plusieurs familles analysées. Les trois plus fréquentes sont 255delA (détectée dans 6 familles) et 202-203delAG (trouvée dans 5 familles) et Arg275Trp (détectée dans 5 familles). Un effet fondateur pourrait être suspecté pour la mutation 255delA qui touche 5 familles françaises. Cependant, cette hypothèse ne pourra être vérifiée que par l'analyse des haplotypes.

### 5. Génétique épidémiologique

Les résultats obtenus montrent que 34 des 77 familles avec un syndrome parkinsonien de début précoce présentent des mutations du gène de la *Parkine,* soulignant l'importance de ce gène dans les familles européennes. La détection de mutations dans 18 des 102 cas isolés et analysés est plus difficile à interpréter car l'effectif est plus petit et l'analyse de certains cas n'est pas complète. Cependant, il est frappant de constater que l'âge de début des 7 cas pour lequel il est connu est particulièrement précoce (13 à 22 ans) et qu'il y a très peu de cas à début très précoce sans mutation du gène de la *Parkine* (par exemple, IT-NA-JMP-3). Ce résultat suggère que la fréquence des mutations du gène de la *Parkine* dans des cas isolés croît lorsque leur âge décroît, spécialement avant l'âge de 25 ans. L'observation de mutations du gène de la *Parkine* dans des cas isolés n'est pas surprenante si l'on considère que dans des familles de petite taille, une maladie autosomique récessive a toutes les chances d'apparaître comme un cas isolé. L'analyse d'un échantillon plus large sera utile pour déterminer précisément la fréquence des mutations de la *Parkine* chez les cas isolés, selon l'âge de début.

Des mutations ont été identifiées dans des familles d'origines très variées : France, Italie, Grande Bretagne, Allemagne, Pays Bas, Algérie, Portugal. Ces résultats montrent que les mutations du gène de la *Parkine* sont mises en évidence dans toutes les populations testées jusqu'à présent.

### 6. Patients avec une anomalie du gène de la Parkine à l'état hétérozygote

Bien que la technique de détection des délétions hétérozygotes d'exons ou des multiplications d'exons nous ait permis d'identifier des cas hétérozygotes composites, dans environ 1/4 des familles (13 sur 53) une seule mutation a été détectée. II s'agit de 6 cas avec une mutation ponctuelle à l'état hétérozygote et de 7 avec une délétion d'exon à l'état hétérozygote. Le rôle pathogène de ces mutations est très probable, car ils causent un changement d'acide aminé non conservatif ou une protéine tronquée. De plus, une de ces mutations de type faux-sens (Arg275Trp) est associée à une autre mutation ponctuelle hétérozygote (Gly430Asp) et à des délétions d'exon hétérozygotes (exon 3-6 ou exon 5+6), portée par l'autre allèle dans trois familles différentes. L'absence de détection d'une mutation sur l'autre allèle dans 13 familles suggère qu'une seconde mutation non détectée affecte une autre région du gène. Cette hypothèse est renforcée par le fait que dans 6 familles probablement liées au locus PARK2, car les patients sont haploidentiques pour 4 marqueurs de la région, aucune mutation n'a été détectée. Ainsi, d'autres régions du gène pourraient être affectées, telles que les régions promotrices, les régions 5' et 3' non traduites, ou des séquences introniques.

### 7. Hétérogénéité génétique des syndromes parkinsoniens autosomiques récessifs de début précoce

Dans 5 des 77 familles, une liaison génétique au locus *Parkine* a pu être exclue. De plus, aucune mutation n'a été identifiée dans 21 familles pour lesquelles ce locus n'a pas pu être formellement exclu. Ces résultats suggèrent qu'il pourrait exister au moins un autre locus pour des familles avec un syndrome parkinsonien autosomique récessif de début précoce en Europe. Cette hypothèse avait été proposée par Leroy et al (1998), qui rapporte une famille avec deux branches, dont l'une présente des délétions du gène de la *Parkine,* alors que l'autre ne présente ni ces délétions ni le même haplotype, ce qui exclu une liaison à ce locus.

### 8. Conclusions

Une nouvelle méthode pour la détection de délétions hétérozygotes d'exons ou des multiplications d'exons est rapportes. En particulier, les duplications/triplications d'exons et des délétions de l'exon 2 et d'autres combinaisons d'exons sont nouvelles. En combinaison avec le séquençage d'exons, huit nouvelles mutations ponctuelles et une délétion intronique qui pourrait affecter un site d'épissage, ont pu être identifiées. Ainsi, 34 des 77 familles analysées (environ 50%) présentent des mutations du gène de la *Paikine.* De plus, les mutations de ce gène ont été détectées dans 19 cas isolés. Dans la population européenne, la proportion de mutations ponctuelles et de délétions d'exons semble identique. Deux points chauds de mutations qui correspondent pour les délétions aux exons 3 à 5 et à trois mutations ponctuelles (202-203delAG, 255delA et Arg275Trp) ont, en outre, été mis en évidence.

### Références bibliographiques

Abbas N, Lücking CB, Ricard S, Dürr A, Bonifati V, De Michele G, Bouley S, Vaughan JR, Gasser T, Marconi R, Broussolle E, Brefel-Courbon C, Harhangi BS, Oostra BA, Fabrizio E, Böhme GA, Pradier L, Wood NW, Filla A, Meco G, Denefle P, Agid Y, Brice A, The French Parkinson's Disease Genetics Study Group and The European Consortium on Genetic Susceptibility in Parkinson's Disease. A wide variety of mutations in the parkin gene are responsible for autosomal recessive parkinsonism in Europe. Hum Mol Genet (1999), 8: 567-574
de Rijk MC, Tzourio C, Bretteler MM, Dartigues JF, Amaducci L, Lopez-Pousa S, Manubens-Bertran JM, Alperovitch A and Rocca WA. Prevalence of parkinsonism and Parkinson's disease in Europe: the EUROPARKINSON Collaborative Study. European Community Concerted Action on the Epidemioiogy of Parkinson's disease. J Neurol Neurosurg Psychiatry (1997), 62: 10-5
Gasser T, MŸller-Myhsok B, Wszolek ZK, Oehlmann R, Calne DB, Bonifati V, Bereznai B, Fabrizio E, Vieregge P and Horstmann RD. A susceptibility locus for Parkinson's disease maps to chromosome 2pl3. Nature Genetics (1998),
Hattori N, Kitada T, Matsumine H, Asakawa S, Yamamura Y, Yoshino H, Kobayashi T, Yokochi M, Wang M, Yoritaka A, Kondo T, Kuznhara S, Nakamura S, Shimizu N and Mizuno Y. Molecular genetic analysis of a novel Parkin gene in Japanese families with autosomal recessive juvenile parkinsonism: evidence for variable homozygous deletions in the Parkin gene in affected individuals. Ann Neurol (1998a), 44: 935-41
Hattori N, Matsumine H, Asakawa S, Kitada T, Yoshino H, Elibol B, Brookes AJ, Yamamura Y, Kobayashi T, Wang M, Yoritaka A, Minoshima S, Shimizu N and Mizuno Y. Point mutations (Thr240Arg and Gln311Stop) [correction of Thr240Arg and Ala311Stop] in the Parkin gene [published erratum appears in Bioehem Biophys Res Commun 1998 Oct 20;251(2):666*].* Biochem Biophys Res Commun (1998b), 249:754-8
Heuegariu O, Heerema NA, Dlouhy SR, Vance GH and Vogt PH. Multiplex PCR: Critical Parameters and Step-by-Step Protocol. BioTechniques (1997),23: 504-511
Ishikawa A and Tsuji S. Clinical analysis of 17 patients in 12 Japanese families with autosomal- recessive type juvenile parkinsonism. Neurology (1996),47: 160-6
Jones AC, Yamamura Y, Almasy L, Bohlega S, Elibol B, Hubble J, Kuzuhara S, Uchida M, Yanagi T, Weeks DE and Nygaard TG. Autosomal Recessive Juvenile Parkinsonism Maps to 6q25.2-q27 in Four Ethnic Groups: Detailed Genetic Mapping of the Linked Region. Am J Hum Genet (1998),63:80-87
Kitada T, Asakawa S, Hattori N, Matsumine H, Yamamura Y, Minoshima S, Yokochi M, Mizuno Y and Shimizu N.Mutations in the parkin gene cause autosomal recessive juvenile parkinsonism [see comments]. Nature (1998), 392: 605-8
Kryger R, Kuhm W, Müller T, Woitalla D, Graeber M, Kssel S, Przuntek H, Epplen JT, Schsls L and Riess O. Ala30Pro mutation in the gene encoding alpha-synuclein in Parkinson's disease. Nature Genetics (1998),18: 106-108
Leroy E, Anastasopoulos D, Konitsiotis S, Lavedan C and Polymeropoulos MH, Deletions in the Parkin gene and genetic heterogeneity in a Greek fami/y with early onset Parkinson's disease. Hum Genet (1998), 103: 424-7
Lücking CB, Abbas N, Dürr A, Bonifati V, Bonnet AM, de Broucker T, De Michele G, Wood NW, Agid Y, Brice A, The European Consortium on Genetic Susceptibility in Parkinson's Disease and The French Parkinson's Disease Genetics Study Group. Homozygous deletions in parkin gene in European and North African families with autosomal recessive juvenile parkinsonism. Lancet (1998), 352: 1355-1356
Matsumine H, Saito M, Shimoda Matsubayashi S, Tanaka H, Ishikawa A, Nakagawa Hattori Y, Yokochi M, Kobayashi T, Igarashi S, Takano H, Sanpei K, Koike R, Mori H, Kondo T, Mizutani Y, Schaffer AA, Yamamura Y, Nakamura S, Kuzuhara S, Tsuji S and Mizuno Y. Localization of a gene for an autosomal recessive form of juvenile Parkinsonism to chromosome 6q25.2-27 Am J Hum Genet (1997), 60: 588-596
Polymeropoulos MH, Lavedan C, Leroy E, Ide SE, Dehejia A, Dutra A, Pike B, Root H, Rubenstein J, Boyer R, Stenroos ES, Chandrasekharappa S, Athanassiadou A, Papapetropoulos T, Johnson WG, Lazzarini AM, Duvoisin RC, Di Iorio G, Golbe LI and Nussbaum RL. Mutations in the alpha-Synuclein Gene Identified in Families with Parkinson's Disease. Science (1997), 276: 2045-2047
Poropat RA and Nicholson GA. Determination of gene dosage at the PMP22 and androgen receptor loci by quantitative PCR. Clinical Chemistry (1998), 44: 724-730
Prior TW. Determining Gene Dosage (editorial). Clinical Chemistry (1998), 44: 703-704
Sunada Y, Saito F, Matsumura K and Shimizu T. Djfferential expression of the parkin gene in the human brain and peripheral leukocytes. Neurosci Lett (1998), 254: 180-182
Takahashi H, Ohama E, Suzuki S, Horikawa Y, Ishikawa A, Morita T, Tsuji S and Ikuta F. Familial juvenile parkinsonism: clinical and pathologic study in a family. Neurology (1994), 44: 437-41
Tassin J, Dürr A, de Broucker T, Abbas N, Bonifati V, De Michele G, Bonnet AM, Broussolle E, Pollak P, Vidailhet M, De Mari M, Marconi R, Medjbeur S, Filla A, Meco G, Agid Y and Brice A. Chromosome 6-Linked Autosomal Recessive Early-Onset Parkinsonism: Linkage in European and Algerian Families, Extension of the Clinical Spectrum, and Evidence of a Small Homozygous Deletion in One Family. Am J Hum Genet (1998), 63: 88-94
Wood N. Genes and parkinsonism [editorial]. J Neurol Neurosurg Psychiatry (1997), 62: 305-9
Yamamura Y, Sobue I, Ando K, lida M and Yanagi T. Paralysis agitans of early onset with marked diurnal fluctuation of symptoms. Neurology (1973),23: 239-44

**TABLEAU 1**

| Position | Changement de nucléotide | Séquence olignucléotidique |
|---|---|---|
| Ex3 | 321-322insGT | WT:5'-TGCAGAGACC--GTGGAGAAAA-3' (SEQ ID N :14) |
| | | V: 5'-GCAGAGACC**GT**GTGGAGAAA-3' (SEQ ID N :15) |
| Ex4 | 584A>T | WT: 5'-GCCGGGAAA**A**CTCAGGGTA-3' (SEQ ID N :16) |
| | | V: 5'-GCCGGGAAA**T**CTCAGGGTA-3' (SEQ ID N :17) |
| Ex7 | 867C>T | WT: 5'-TGCAACTCC**C**GCCACGTGA-3' (SEQ ID N :18) |
| | | V: 5'-TGCAACTCC**T**GCCACGTGA-3' (SEQ ID N :19) |
| Ex10 | 1239G>C | WT: 5'-TGCAGTGCC**G**TATTTGAAG-3' (SEQ ID N :20) |
| | | V: 5'-TGCAGTGCC**C**TATTTGAAG-3' (SEQ ID N :21) |
| Ex11 | 1345 C>A | WT: 5'-AGAAAACCA**C**CAAGCCCTG-3' (SEQ ID N :22) |
| | | V: 5'-AGAAAACCAACA**A**GCCCTG-3' (SEQ ID N :23) |

**TABLEAU 2**

| | **nucléotide changé** | **amino acide changé/ position du codon stop** | **type de mutation** | **technique de détection** | **longueur attendue du fragment en bp** |
|---|---|---|---|---|---|
| ***exonique*** | | | | | |
| Ex2 | ***202-203delAG*** | ***Gln34Arg(Stop37)*** | cadre de lecture | PAGE | WT: 308 V:306 |
| Ex2 | ***255delA*** | ***Asn52Met(Stop81)*** | cadre de lecture | *Fok* I création de site | WT: 278+30 V:222+57+30 |
| Ex3 | ***321-322insGT*** | ***Trp74Cys(Stop81)*** | cadre de lecture | ASO | |
| | | | | | |
| Ex4 | ***584A*>*T*** | ***Lys161Asn*** | faux-sens (non conservative) | ASO | |
| Ex4 | 601G>A | Ser167Asn | faux-sens (conservative) | *Alw*N I perte du site | WT: 164+97 V: 261 |
| **Ex6** | ***734A*>*T*** | ***Lys211Asn*** | **faux-sens (non conservative)** | ***Dra* I perte du site** | **WT: 171+98 V: 269** |
| Ex7 | ***867C*>*T*** | ***Arg256Cys*** | faux-sens (non conservative) | ASO | |
| **Ex7** | ***905T*>*A*** | ***Cys268Stop*** | **non-sens** | ***Dde* I gain de site** | **WT: 141+100 V: 117+100+24** |
| Ex7 | ***924C*>*T*** | ***Arg275Trp*** | faux-sens (non conservative) | *Sau*3A I perte du site | WT: 142+97 V: 239 |
| **Ex7** | ***939G>A*** | ***Asp280Asn*** | **faux-sens (non conservative)** | ***Alw* I avec amorce à mismatch perte du site** | **WT: 153+30 V: 183** |
| **Ex7** | ***966T>G*** | ***Cys289Gly*** | **faux-sens (conservative)** | ***BstN* I gain** de site | **WT: 177+64 V: 118+64+59** |
| **Ex 9** | ***1142-1143delGA*** | ***Arg348Glu(Stop368)*** | **cadre de lecture** | ***PAGE*** | **WT: 278 V: 276** |
| **Ex9** | ***1084G*>*A*** | ***Gly328Glu*** | **faux-sens (non conservative)** | ***Mnl* I gain de site** | **WT: 124+80+74 V:124+74+60+20** |
| **Ex9** | ***1101C*>*T*** | ***Arg334Cys*** | **fanx-sens (non conservative)** | ***BstU* I avec amorce à mismatch perte du site** | **WT: 123+21 V:144** |
| Ex10 | 1239G>C | Val380Leu | faux-sens (conservative) | ASO | |
| Ex11 | 1281G>A | Asp394Asn | faux-sens (non conservative) | *Taq* I perte du site | WT: 221+84 V: 303 |
| Ex11 | ***1345C*>*A*** | ***Thr415Asn*** | faux-sens (conservative) | ASO | |
| **Ex12** | ***1390G*>*A*** | ***Gly430Asp*** | **faux-sens (non conservative)** | ***Mnl* I perte du site** | **WT: 191+65 V: 256** |
| Ex12 | ***1459G*>*A*** | ***Trp453Stop*** | non-sens | *Nla* IV perte du site | WT: 142+17+35+61 V: 159+35+61 |
| ***intronique*** | | | | | |
| Intron 2 | IVS2+25T>C (272+25T>C) | | | *Bst*N I création de site | WT: 308 bp V: 264+44 |
| Intron 3 | IVS3-20C>T (514-20C>T) | | | *Mnl* I perte du site | WT: 201+60 V: 261 |
| Intron 7 | IVS7-35A>G (973-35A>G) | | | *Mae* III création de site | WT: 206 V:159+47 |
| **Intron** 8 | **IVS8-21-17del (1035-21-17del) TCTGC** | **splice site** | | **PAGE** | |

**TABLEAU 3 CARACTÉRISTIQUES CLINIQUES DES FAMILLESAVEC MUTATIONS DU GÈNE DE LA PARKINE**

| | Déplétions homozygotes | Mutations faux sens | Mutations tronquantes | Total |
|---|---|---|---|---|
| Familles (Patients) | 3(8) | 3(8) | 4(9) | 10(25) |
| Age moyen de début (extrêmes) | 30 ± 16 (7-55) | 44 ± 9 (30-56) | 37±6 (29-45) | 37±12(7-56) |
| Durée moyenne d'évolution (extrêmes) | 13 ± 6 (3-20) | 13 ± 7 (0.5-27) | 16±10 (3-29) | 14±8(0,5-29) |
| Echelle de Hoehn et Yahr | 3.1 ± 1.2 | 2.6 ± 0.8 | 2.0±0.6 | 2,5±0,98 |
| Akinésie | 8/8 | 8/8 | 8/9 | 96% |
| Rigidité | 8/8 | 8/8 | 9/9 | 100% |
| Tremblement | 3/8 | 4/8 | 8/9 | 60% |
| Dystonie | 4/8 | 0/5 | 1/7 | 25% |
| Bonne réactivité à la levodopa (cas de novo) | 77 (1) | 6/6 (2) | 7/7 | 100% |
| Dyskinésies | 4/7 | 5/6 | 6/9 | 68% |
| Fluctuations^{a} | 7/7 | ND | 2/6 | 62% |
| Réflexes vifs aux MI | 2/8 | 3/4 | 0/6 | 28% |

**TABLEAU 4**

| **exon(s) délété(s)/multiplié(s)** | **nombre de familles** | **conséquences** |
|---|---|---|
| **2 del** | **3 x het** | **Décalage du cadre de lecture** |
| **2 triplication** | **1x hom + 1x het** | **Pas de décalage du cadre de lecture** |
| **2+3 del** | **1 x het** | **Pas de décalage du cadre de lecture** |
| **2+3+4 del** | **1 x het** | **Décalage du cadre de lecture** |
| **3 del** | **3 x hom + 7 x het** | **Décalage du cadre de lecture** |
| **3 duplication** | **1x hom + 1x het** | **Décalage du cadre de lecture** |
| **3+4 del** | **1 x hom + 3 x het** | **Pas de décalage du cadre de lecture** |
| **3-6 del** | **1x het** | **Décalage du cadre de lecture** |
| **3-9 del** | **1 x het** | **Pas de décalage du cadre de lecture** |
| **4 del** | **1 x hom + 3 x het** | **Décalage du cadre de lecture** |
| **5 del** | **3 x het** | **Pas de décalage du cadre de lecture** |
| **5+6 del** | **2 x hom** | **Décalage du cadre de lecture** |
| **6 del** | **1 x het** | **Décalage du cadre de lecture** |
| **6 duplication** | **1x het** | **Décalage du cadre de lecture** |
| **6+7 del** | **1x het** | **Décalage du cadre de lecture** |
| **7 duplication** | **1x het** | **Décalage du cadre de lecture** |
| **7+8+9 del** | **1 x het** | **Décalage du cadre de lecture** |
| **8 del** | **1 x het** | **Décalage du cadre de lecture** |
| **8+9 del** | **1 x hom** | **Décalage du cadre de lecture** |
| **11 duplication** | **1x het** | **Décalage du cadre de lecture** |

**TABLEAU 5**

| ***cas*** | ***3i*** | ***12*** | ***9*** | ***2*** | ***C328*** | ***C328*/*3i*** | ***C328*/*12*** | ***C328*/*9*** | ***C328*/*2*** | ***Ex3i*/*12*** | ***Ex3i*/*9*** | ***Ex3i*/*2*** | ***Ex12*/*9*** | ***Ex12*/*2*** | ***Ex9*/*2*** |
|---|---|---|---|---|---|---|---|---|---|---|---|---|---|---|---|
| **T2** | **743** | **838** | **1040** | **935** | **455** | **0.61** | **0.54** | **0.44** | **0.49** | **0.89** | **0.71** | **0.79** | **0.81** | **0.90** | **1.11** |
| **FR 155 5** T2/FR 155 5 | **608** | **1245** | **1588** | **1466** | **635** | **1.04** 0.59 | ***0.51** 1.06* | ***0.40** 1.09* | ***0.43** 1.12* | **0.49** 1.82 | **0.38** 1.87 | **0.41** 1.92 | ***0.78** 1.03* | ***0.85*** *1.06* | ***1.08** 1.03* |
| **FR 155 6** T2/FR 156 6 | **759** | **861** | **1120** | **540** | **498** | ***0.66** 0.93* | ***0.58** 0.94* | ***0.44** 0.98* | **0.92** 0.53 | ***0.88*** *1.01* | ***0.68** 1.05* | **1.41** 0.57 | ***0.77*** *1.05* | **1.59** 0.56 | **2.07** 0.54 |
| **FR 155 8** T2/FR 155 8 | **597** | **1185** | **1467** | **766** | **623** | **1.04** 0.59 | ***0.53** 1.03* | ***0.42** 1.03* | **0.81** 0.60 | **0.50** 1.76 | 0.411.76 | ***0.78*** *1.02* | ***0.81** 1.00* | **1.55** 0.58 | **1.92** 0.58 |
| **FR 155 9** T2/FR 155 9 | **495** | **1200** | **1438** | **754** | **688** | **1.39 0.44** | ***0.57*** *0.95* | ***0.48** 0.91* | **0.91** 0.53 | **0.41** 2.15 | 0.34 2.08 | ***0.66*** *1.21* | ***0.83** 0.97* | **1.59** 0.56 | **1.91** 0.58 |

## Revendications

1. Acide nucléique codant pour la parkine humaine, **caractérisé en ce qu**'il comporte 1a délétion des exons 2 et 3.

2. Polypeptide codé par un acide nucléique selon la revendication 1.

3. Anticorps spécifique d'un polypeptide selon la revendication 2.

4. Composition comprenant un polypeptide selon la revendication 2 ou un anticorps selon la revendication 3.

5. Sonde nucléotidique **caractérisée en ce qu**'elle hybride spécifiquement avec un acide nucléique selon la revendication 1.

6. Procédé pour l'identification d'une altération génétique telle que la déletion des exons 2 et 3 dans le gène de la parkine comprenant:
i) la mise a disposition d'un échantillon test comprenant le gène de la parkine,
ii) l'amplification de la partie dudit gène comprenant potentiellement ladite altération et
iii) la mise en évidence de la présence de l'altération génétique.

7. Procédé selon la revendication 6, **caractérisé en ce que** l'échantillon est un échantillon de sang, tissu, plasma ou une culture de cellules.

8. Procédé selon la revendication 7, **caractérisé en ce que** l'échantillon est prétraité de manière à rendre le gène de la parkine, ou une partie de celui-ci, accessible pour l'amplification.

9. Procédé selon la revendication 6, **caractérisé en ce que** l'amplification est réalisée au moyen d'un couple d'amorces pour l'amplification de tout ou partie d'un acide nucléique tel que décrit selon la revendication 1, **caractérisé en ce qu**'il comprend :
i) une première amorce complémentaire d'une région du gène de la parkine située en 5' de ladite mutation, et
ii) une deuxième amorce complémentaire d'une région du gène de la parkine située en 3' de ladite mutation.

10. Procédé selon la revendication 6, **caractérisé en ce que** la mise en évidence est réalisée par séquençage, PCR/restriction, ASO, PAGE, ou PCR/multiplexe semi-quantitative.

11. Procédé de diagnostique d'une susceptibilité au syndrome parkinsonien comprenant la recherche d'une altération génétique dans le gène de la parkine selon la revendication 1.

12. Vecteur comprenant un acide nucléique tel que décrit selon la revendication 1.

13. Cellule comprenant un vecteur tel que décrit selon la revendication 12.

14. Mammifère non-humain comprenant, dans ses cellules, un acide nucléique tel que décrit selon la revendication 1.

15. Utilisation d'une cellule selon la revendication 13 ou d'un mammifère selon la revendication 14 pour l'identification de composés capables de contrecarrer les effets d'une altération génétique du gène de la parkine.


LISTE DE SEQUENCES

<110> RHONE POULENC RORER
      INSTITUT NATIONAL DE LA SANTE ET DE LA RECHERCHE M

<120> MUTATIONS DU GENE DE LA PARKIN, COMPOSITIONS, METHODES
      ET UITLISATIONS

<130> parkin

<140>
<141>

<150> FR9814254
<151> 1998-11-19

<150> FR9910140
<151> 1999-08-04

<150> US60124239
<151> 1999-03-12

<160> 23

<170> PatentIn Ver. 2.1

<210> 1
<211> 2960
<212> ADN
<213> Homo sapiens

<400> 1
   tccgggagga ttacccagga gaccgctggt gggaggcgcg gctggcgccg ctgcgcgcat 60
   gggcctgttc ctggcccgca gccgccacct acccagtgac catgatagtg tttgtcaggt 120
   tcaactccag ccatggtttc ccagtggagg tcgattctga caccagcatc ttccagctca 180
   aggaggtggt tgctaagcga cagggggttc cggctgacca gttgcgtgtg attttcgcag 240
   ggaaggagct gaggaatgac tggactgtgc agaattgtga cctggatcag cagagcattg 300
   ttcacattgt gcagagaccg tggagaaaag gtcaagaaat gaatgcaact ggaggcgacg 360
   accccagaaa cgcggcggga ggctgtgagc gggagcccca gagcttgact cgggtggacc 420
   tcagcagctc agtcctccca ggagactctg tggggctggc tgtcattctg cacactgaca 480
   gcaggaagga ctcaccacca gctggaagtc cagcaggtag atcaatctac aacagctttt 540
   atgtgtattg caaaggcccc tgtcaaagag tgcagccggg aaaactcagg gtacagtgca 600
   gcacctgcag gcaggcaacg ctcaccttga cccagggtcc atcttgctgg gatgatgttt 660
   taattccaaa ccggatgagt ggtgaatgcc aatccccaca ctgccctggg actagtgcag 720
   aatttttctt taaatgtgga gcacacccca cctctgacaa ggaaacatca gtagctttgc 780
   acctgatcgc aacaaatagt cggaacatca cttgcattac gtgcacagac gtcaggagcc 840
   ccgtcctggt tttccagtgc aactcccgcc acgtgatttg cttagactgt ttccacttat 900
   actgtgtgac aagactcaat gatcggcagt ttgttcacga ccctcaactt ggctactccc 960
   tgccttgtgt ggctggctgt cccaactcct tgattaaaga gctccatcac ttcaggattc 1020
   tgggagaaga gcagtacaac cggtaccagc agtatggtgc agaggagtgt gtcctgcaga 1080
   tggggggcgt gttatgcccc cgccctggct gtggagcggg gctgctgccg gagcctgacc 1140
   agaggaaagt cacctgcgaa gggggcaatg gcctgggctg tgggtttgcc ttctgccggg 1200
   aatgtaaaga agcgtaccat gaaggggagt gcagtgccgt atttgaagcc tcaggaacaa 1260
   ctactcaggc ctacagagtc gatgaaagag ccgccgagca ggctcgttgg gaagcagcct 1320
   ccaaagaaac catcaagaaa accaccaagc cctgtccccg ctgccatgta ccagtggaaa 1380
   aaaatggagg ctgcatgcac atgaagtgtc cgcagcccca gtgcaggctc gagtggtgct 1440
   ggaactgtgg ctgcgagtgg aaccgcgtct gcatggggga ccactggttc gacgtgtagc 1500
   cagggcggcc gggcgcccca tcgccacatc ctgggggagc atacccagtg tctaccttca 1560
   ttttctaatt ctcttttcaa acacacacac acacgcgcgc gcgcgcacac acactcttca 1620
   agtttttttc aaagtccaac tacagccaaa ttgcagaaga aactcctgga tccctttcac 1680
   tatgtccatg aaaaacagca gagtaaaatt acagaagaag ctcctgaatc cctttcagtt 1740
   tgtccacaca agacagcaga gccatctgcg acaccaccaa caggcgttct cagcctccgg 1800
   atgacacaaa taccagagca cagattcaag tgcaatccat gtatctgtat gggtcattct 1860
   cacctgaatt cgagacaggc agaatcagta gctggagaga gagttctcac atttaatatc 1920
   ctgcctttta ccttcagtaa acaccatgaa gatgccattg acaaggtgtt tctctgtaaa 1980
   atgaactgca gtgggttctc caaactagat tcatggcttt aacagtaatg ttcttattta 2040
   aattttcaga aagcatctat tcccaaagaa ccccaggcaa tagtcaaaaa catttgttta 2100
   tccttaagaa ttccatctat ataaatcgca ttaatcgaaa taccaactat gtgtaaatca 2160
   acttgtcaca aagtgagaaa ttatgaaagt taatttgaat gttgaatgtt tgaattacag 2220
   ggaagaaatc aagttaatgt actttcattc cctttcatga tttgcaactt tagaaagaaa 2280
   ttgtttttct gaaagtatca ccaaaaaatc tatagtttga ttctgagtat tcattttgca 2340
   acttggagat tttgctaata catttggctc cactgtaaat ttaatagata aagtgcctat 2400
   aaaggaaaca cgtttagaaa tgatttcaaa atgatattca atcttaacaa aagtgaacat 2460
   tattaaatca gaatctttaa agaggagcct ttccagaact accaaaatga agacacgccc 2520
   gactctctcc atcagaaggg tttatacccc tttggcacac cctctctgtc caatctgcaa 2580
   gtcccaggga gctctgcata ccaggggttc cccaggagag accttctctt aggacagtaa 2640
   actcactaga atattcctta tgttgacatg gattggattt cagttcaatc aaactttcag 2700
   cttttttttc agccattcac aacacaatca aaagattaac aacactgcat gcggcaaacc 2760
   gcatgctctt acccacacta cgcagaagag aaagtacaac cactatcttt tgttctacct 2820
   gtattgtctg acttctcagg aagatcgtga acataactga gggcatgagt ctcactagca 2880
   catggaggcc cttttggatt tagagactgt aaattattaa atcggcaaca gggcttctct 2940
   ttttagatgt agcactgaaa 2960

<210> 2
<211> 19
<212> ADN
<213> Homo sapiens

<400> 2
aattgtgacc tggatcagc 19

<210> 3
<211> 23
<212> ADN

<213> Homo sapiens

<400> 3
ctggacttcc agctggtggt gag                                              23


<210> 4
<211> 25
<212> ADN
<213> Homo sapiens

<400> 4
ggcagggagt agccaagttg aggat                                            25


<210> 5
<211> 22
<212> ADN
<213> Homo sapiens

<400> 5
agccccgctc cacagccagc gc                                               22


<210> 6
<211> 19
<212> ADN
<213> Homo sapiens

<400> 6
aattgtgacc tggatcagc                                                   19


<210> 7
<211> 23
<212> ADN
<213> Homo sapiens

<400> 7
ctggacttcc agctggtggt gag                                              23


<210> 8
<211> 19
<212> ADN
<213> Homo sapiens

<400> 8

aattgtgacc tggatcagc 19

<210> 9
<211> 23
<212> ADN
<213> Homo sapiens

<400> 9
ctggacttcc agctggtggt gag 23

<210> 10
<211> 20
<212> ADN
<213> Homo sapiens

<400> 10
acgttcctga taatgggatc 20

<210> 11
<211> 20
<212> ADN
<213> Homo sapiens

<400> 11
cctctctcta ccaagtgagg 20

<210> 12
<211> 25
<212> ADN
<213> Homo sapiens

<400> 12
ggcagggagt agccaagttg aggat 25

<210> 13
<211> 22
<212> ADN
<213> Homo sapiens

<400> 13
agccccgctc cacagccagc gc 22

<210> 14
<211> 20
<212> ADN
<213> Homo sapiens

<400> 14
tgcagagacc gtggagaaaa                                                    20

<210> 15
<211> 20
<212> ADN
<213> Homo sapiens

<400> 15
gcagagaccg tgtggagaaa                                                    20

<210> 16
<211> 19
<212> ADN
<213> Homo sapiens

<400> 16
gccgggaaaa ctcagggta                                                     19

<210> 17
<211> 19
<212> ADN
<213> Homo sapiens

<400> 17
gccgggaaat ctcagggta                                                     19

<210> 18
<211> 19
<212> ADN
<213> Homo sapiens

<400> 18
tgcaactccc gccacgtga                                                     19

<210> 19
<211> 19
<212> ADN

<213> Homo sapiens

<400> 19
tgcaactcct gccacgtga                                                    19

<210> 20
<211> 19
<212> ADN
<213> Homo sapiens

<400> 20
tgcagtgccg tatttgaag                                                    19

<210> 21
<211> 19
<212> ADN
<213> Homo sapiens

<400> 21
tgcagtgccc tatttgaag                                                    19

<210> 22
<211> 19
<212> ADN
<213> Homo sapiens

<400> 22
agaaaaccac caagccctg                                                    19

<210> 23
<211> 19
<212> ADN
<213> Homo sapiens

<400> 23
agaaaaccaa caagccctg                                                    19